# EUROPEAN PATENT APPLICATION

(11) **EP 4 344 615 A1**
(43) Date of publication of application: **03.04.2024**
(21) Application number: 23199578.8
(22) Date of filing: 26.09.2023
(51) Int. Cl.: A61B 3/103

(54) **OPHTHALMOLOGIC APPARATUS**

(30) Priority: 29.09.2022 JP 2022157139; 16.06.2023 JP 2023099210
(71) Applicant: TOPCON CORPORATION, Tokyo 174-8580 (JP)
(72) Inventor: YUKIMORI, Takafumi, Tokyo, 174-8580 (JP); TATARA, Yoko, Tokyo, 174-8580 (JP); SAIKA, Makoto, Tokyo, 174-8580 (JP)
(74) Representative: Louis Pöhlau Lohrentz

(57) **Abstract**

An ophthalmologic apparatus includes a target projection system (140) that presents a target to a subject eye (E), an objective measurement optical system (160, 170) that objectively measures an eye characteristic of the subject eye (E), and a control portion (40) that controls the target projection system (140) and the objective measurement optical system (160, 170), wherein the control portion (40) presents the target for a predetermined time at a predetermined presentation position with the target projection system (140), and objectively measures the eye characteristic with the objective measurement optical system (160, 170) in a time series to control an operation of each portion based on a measurement result of the obj ective measurement.

## Description

### FIELD OF THE INVENTION

The present disclosure relates to an ophthalmologic apparatus.

### BACKGROUND

An ophthalmologic apparatus that accelerates recovery of eyestrain (see e.g., JP2007-215691A) by training recovery of visual acuity for depth of a patient with binocular parallax, and also an ophthalmologic apparatus that accelerates recovery of eyestrain by being stimulated for inducing pupil reaction while checking pupil reaction by a patient (see e.g., JP2005-279053A) are conventionally known.

### SUMMARY

An eye characteristic such as refractive power and pupil reaction of a subject eye is changed by being stimulated to induce visual recognition of a target and pupil reaction. However, in the conventional ophthalmologic apparatus, the condition of the subject eye changed by being stimulated could not be reflected to the operation control of the ophthalmologic apparatus, and the operation control of the ophthalmologic apparatus could not be automatically performed based on the eye characteristic of the subject eye after being stimulated.

The present disclosure has been made in view of the above problem, and an object of the present disclosure is to provide an ophthalmologic apparatus capable of automatically performing operation control according to a condition of a subject eye after visually recognizing a target.

To achieve the above object, an ophthalmologic apparatus includes a target projection system that presents a target to a subject eye, an objective measurement optical system that objectively measures an eye characteristic of the subject eye, and a control portion that controls the target projection system and the objective measurement optical system, wherein the control portion presents the target for a predetermined time at a predetermined presentation position with the target projection system, and objectively measures the eye characteristic with the objective measurement optical system in a time series to control an operation of each portion based on a measurement result of the objective measurement.

### EFFECT OF THE INVENTION

According to the ophthalmologic apparatus of the present disclosure as configured above, the operation can be automatically controlled according to the condition of the subject eye after visually recognizing the target.

### BRIEF DESCRIPTION OF DRAWINGS

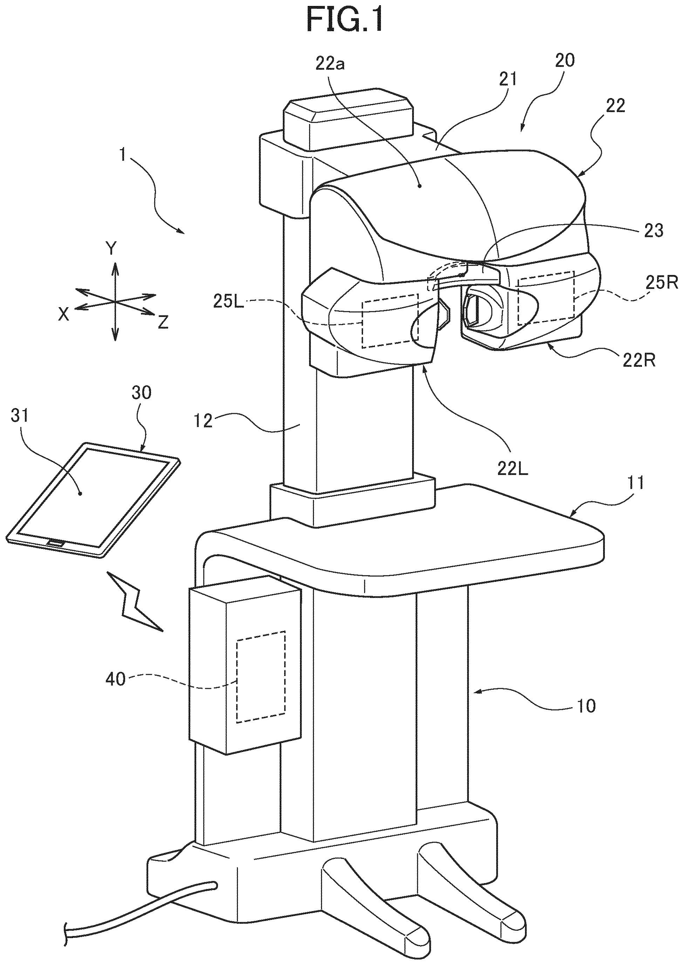

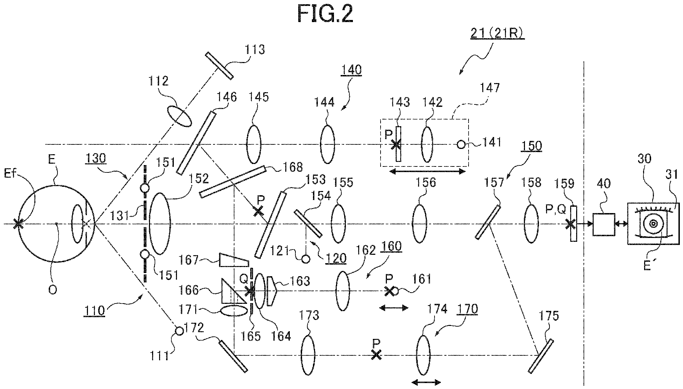

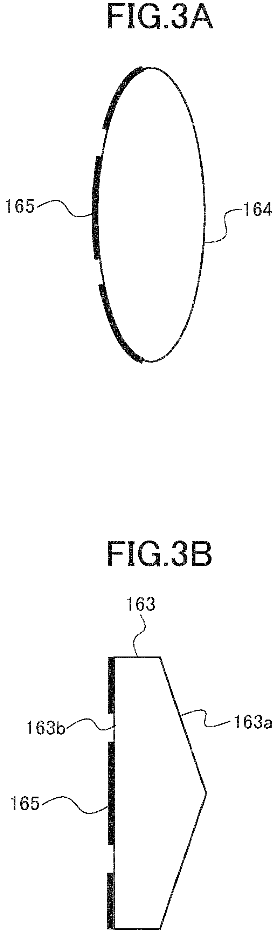

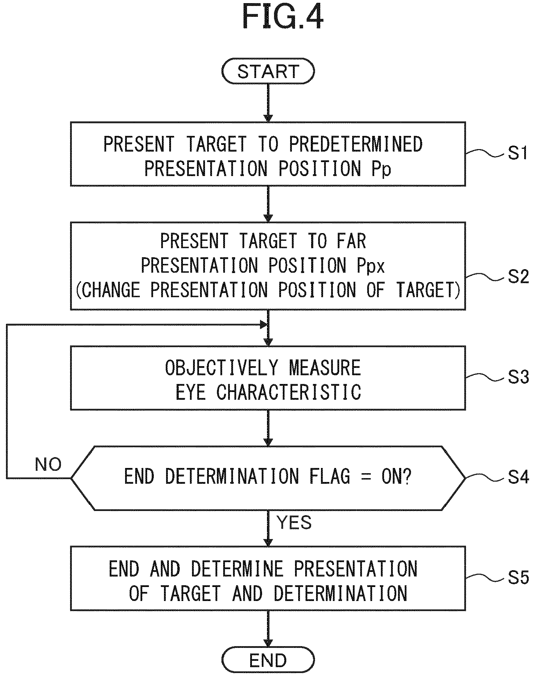

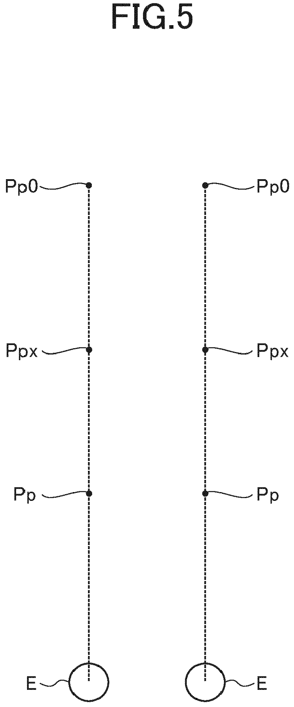

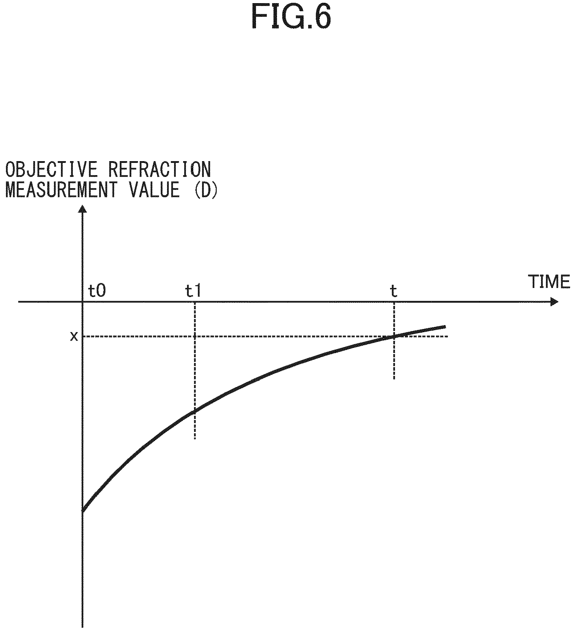

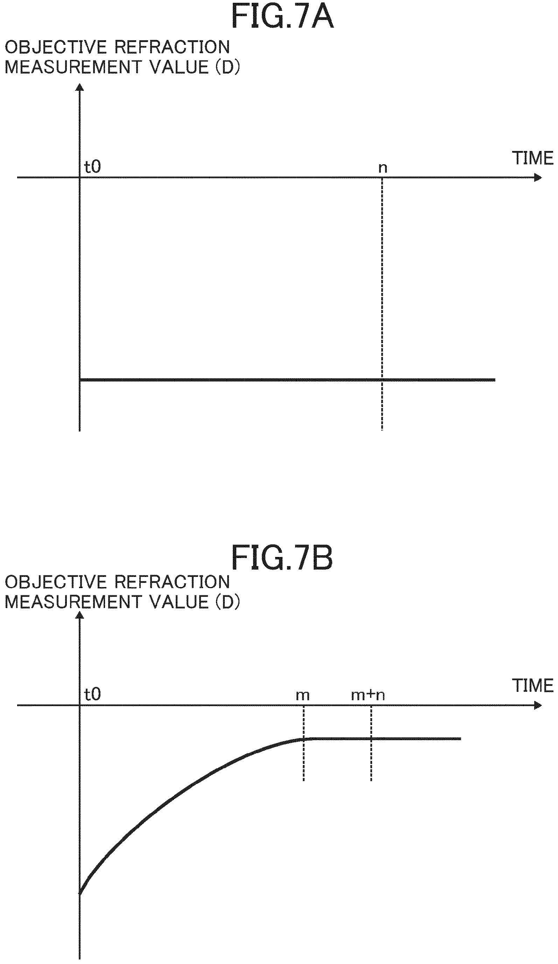

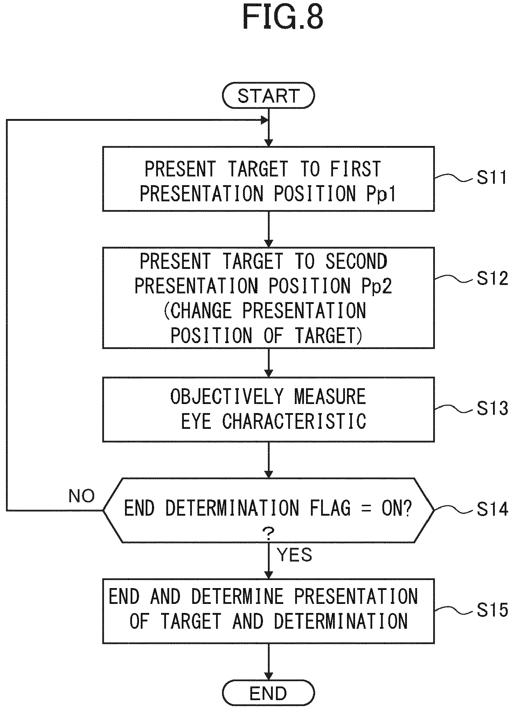

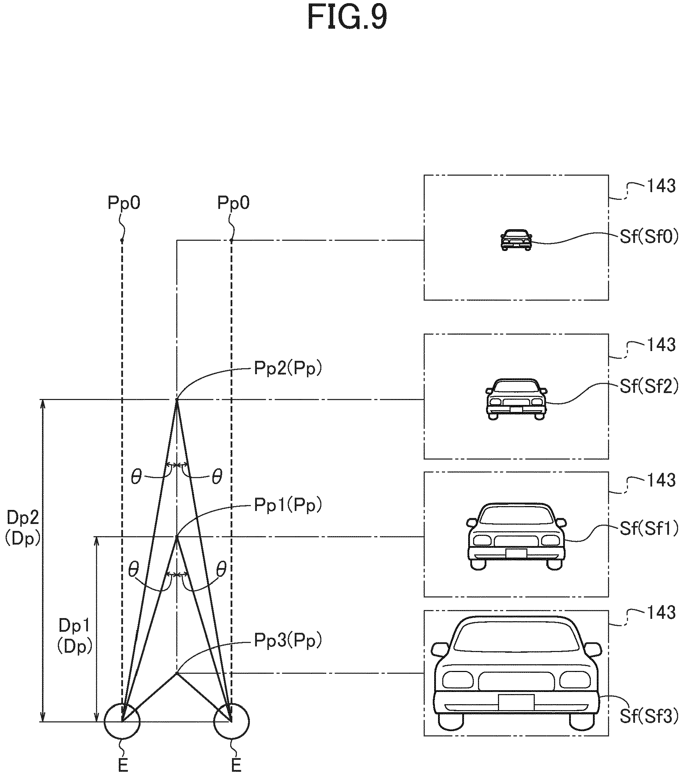

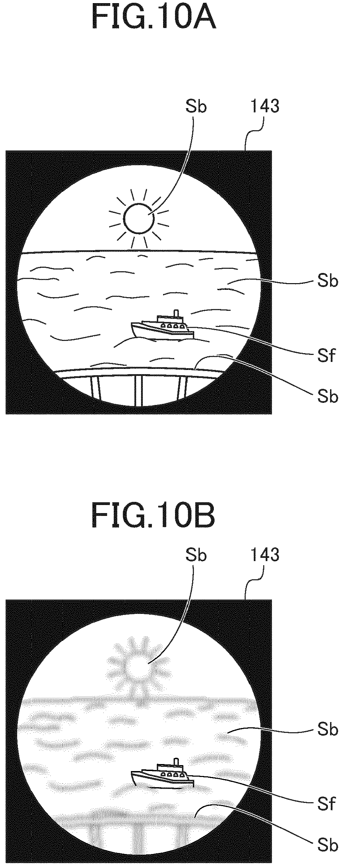

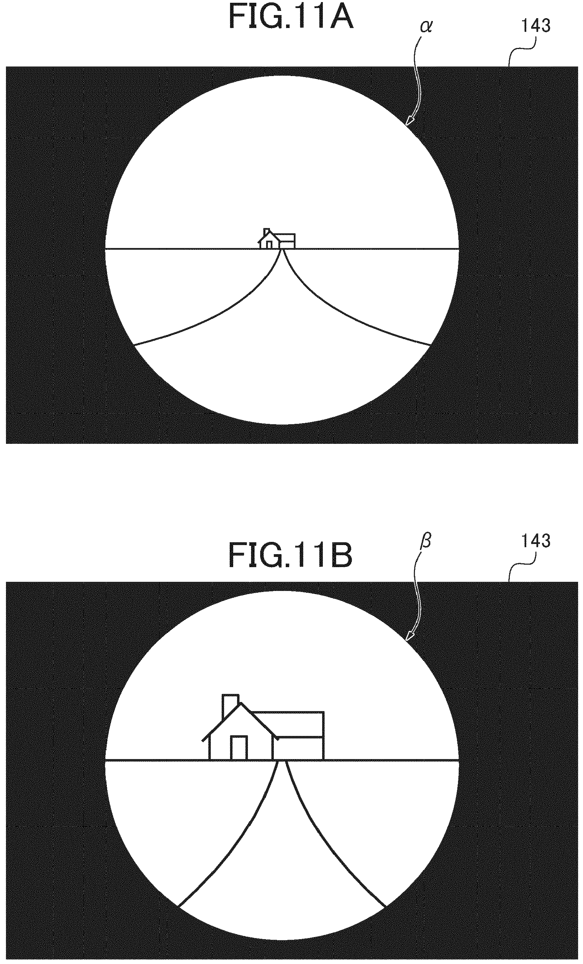

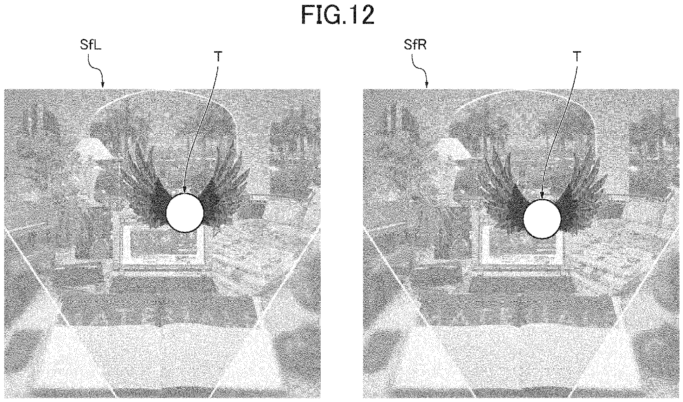

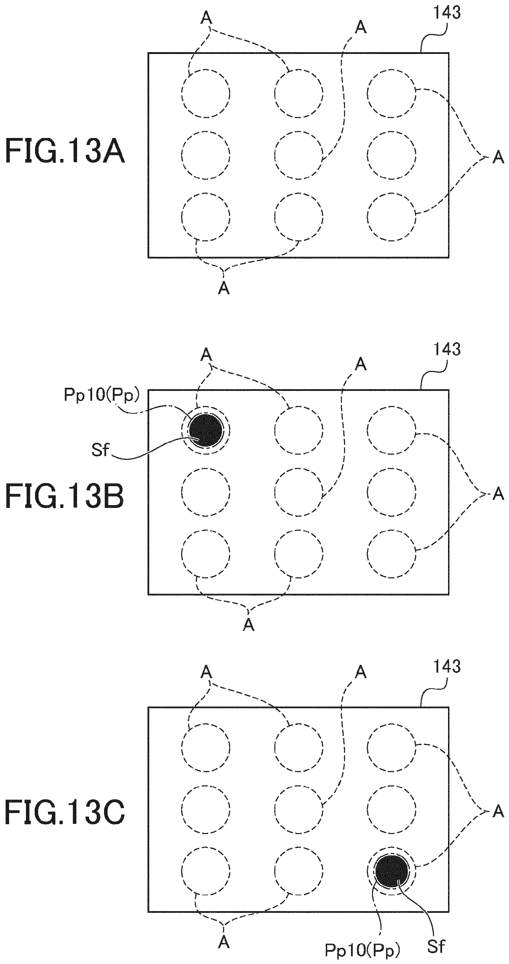

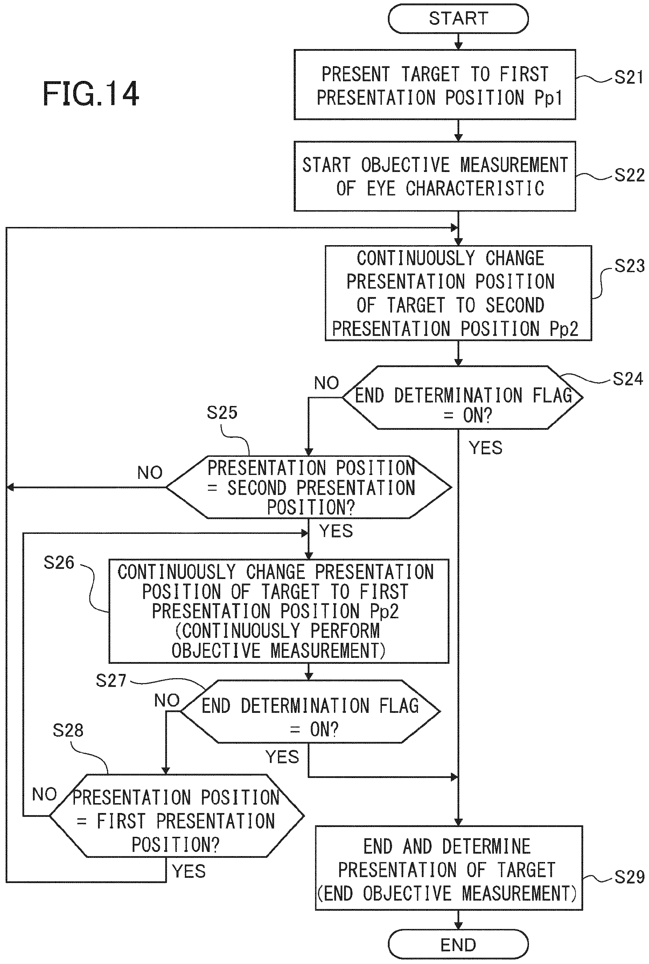

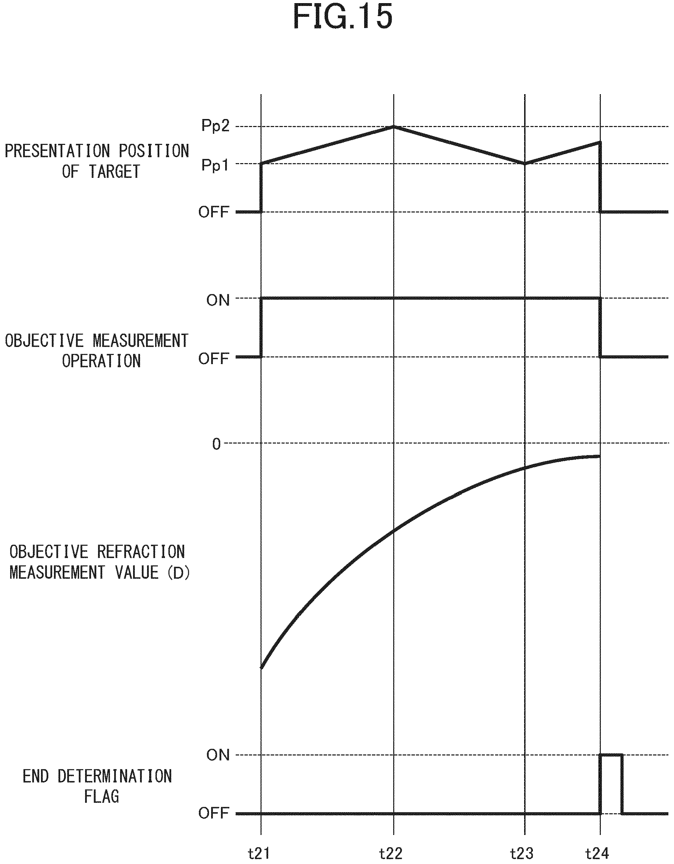

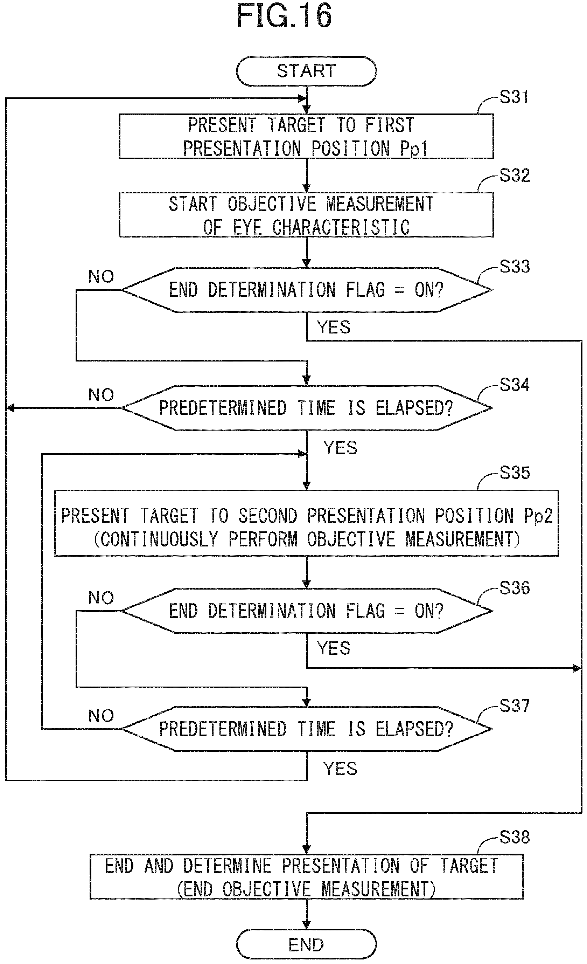

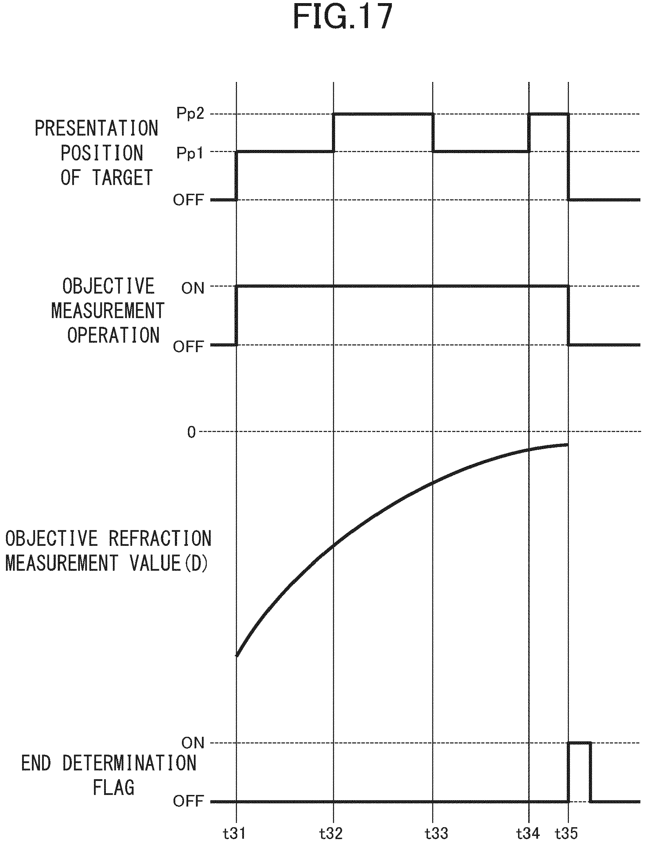

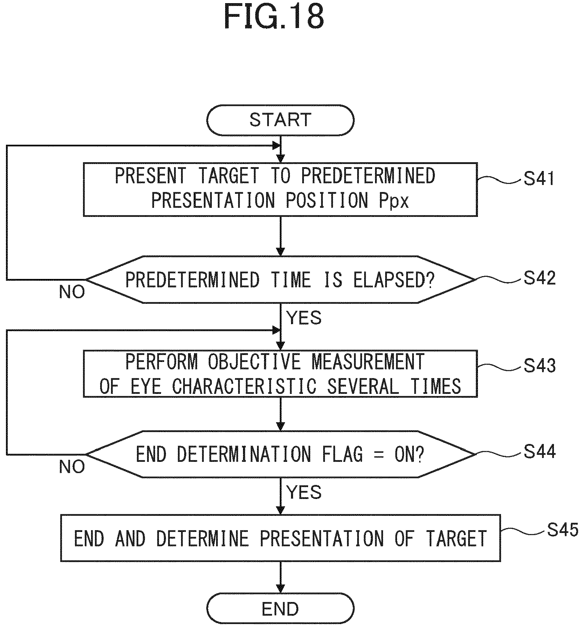

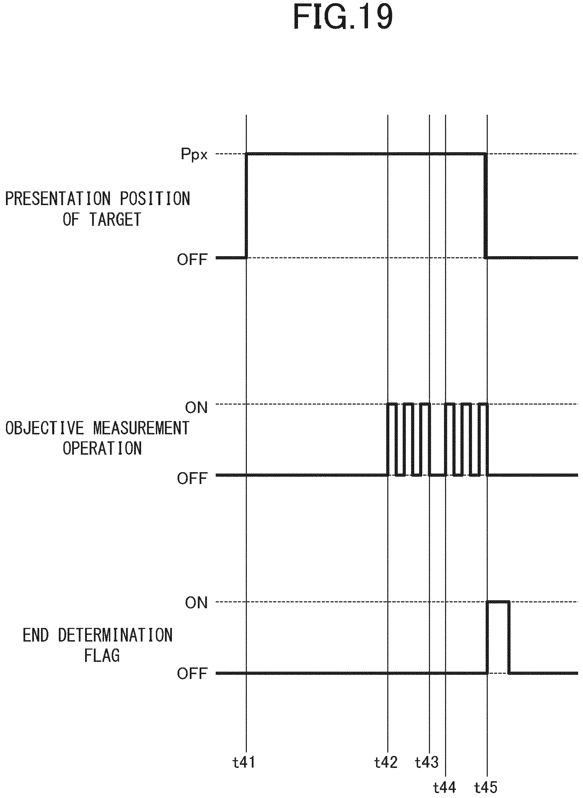

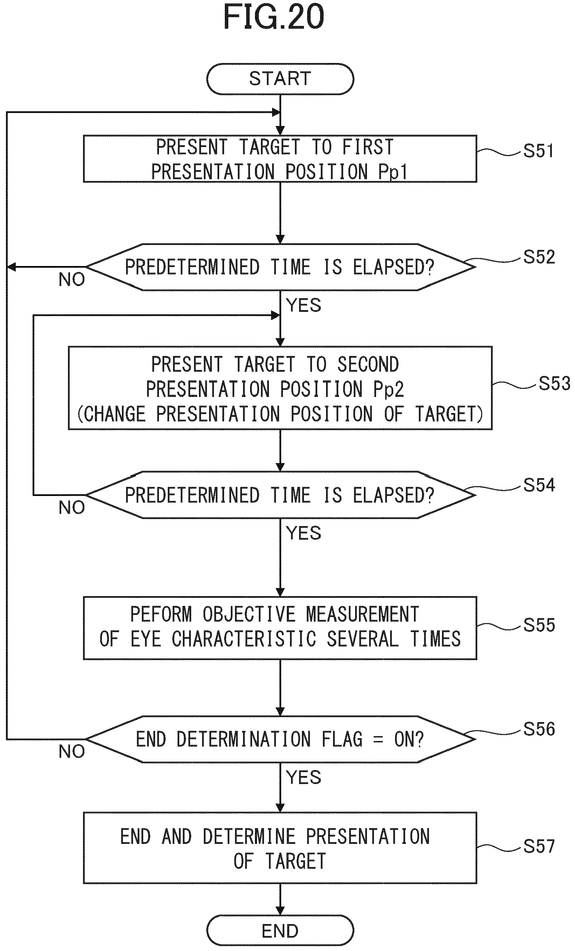

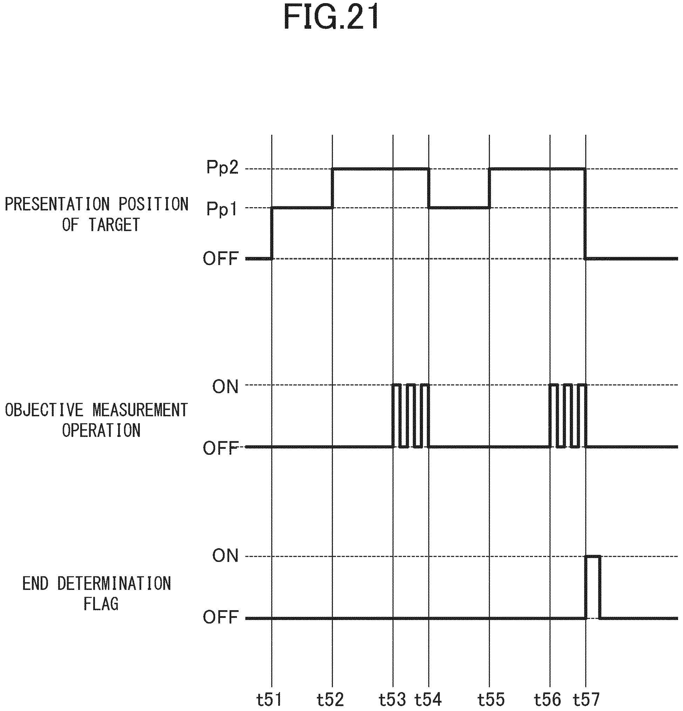

FIG. 1 is a perspective view illustrating an entire configuration of an ophthalmologic apparatus of a first embodiment. FIG. 2 is a view illustrating a detailed configuration of a left measurement optical system of the ophthalmologic apparatus of the first embodiment. FIG. 3A is a schematic view illustrating a configuration example of a field lens for use in the ophthalmologic apparatus of the first embodiment. FIG. 3B is a schematic view illustrating a configuration example of a cone prism for use in the ophthalmologic apparatus of the first embodiment. FIG. 4 is a flowchart illustrating a flow of control that is performed by a control portion of the first embodiment. FIG. 5 is a drawing describing a presentation position of a target in the first embodiment. FIG. 6 shows a first example of a graph showing a measurement result of objective measurement. FIG. 7A shows a second example of a graph showing a measurement result of objective measurement. FIG. 7B shows a third example of a graph showing a measurement result of objective measurement. FIG. 8 is a flowchart illustrating a flow of control that is performed by a control portion of a second embodiment. FIG. 9 is a drawing describing a presentation position of a target in the second embodiment. FIG. 10A is a drawing illustrating an example of a target having a background image that is presented to a subject eye. FIG. 10B is a drawing illustrating an example of a target having an unclear background image that is presented to a subject eye. FIG. 11A is a drawing illustrating an example of a wide-angle image that is presented to a subject eye. FIG. 11B is a drawing illustrating an example of a narrow-angle image that is presented to a subject eye. FIG. 12 is a view illustrating an example of a target to which parallax is given, and the target being presented to a subject eye. FIG. 13Ais a drawing illustrating a target presentation region that is set to an eye chart. FIG. 13B is a drawing illustrating an example in which a target is illustrated in a predetermined target presentation region. FIG. 13C is a drawing illustrating an example in which a target is presented to a target presentation region different from the region illustrated in FIG. 3B. FIG. 14 is a flowchart of the first example showing a flow of control in objective measurement during presenting a target. FIG. 15 is a time chart showing a relationship among a presentation position controlled along the flowchart of FIG. 14, an objective refraction measurement operation, an objective measurement value, and an end determination flag. FIG. 16 is a flowchart of a second example showing flow of control when objective measurement is performed during presenting a target. FIG. 17 is a time chart showing a relationship among a presentation position of a target controlled along the flowchart of FIG. 16, objective measurement operation, an objective refraction measurement value, and an end determination flag. FIG. 18 is a flowchart of a first example showing a flow of control when an objective measurement is performed several times a target is presented. FIG. 19 is a time chart showing a relationship among a presentation position of a target controlled along the flowchart of FIG. 18, objective measurement operation, and an end determination flag. FIG. 20 is a flowchart of a second example showing a flow of control when objective measurement is performed several times after a target is presented. FIG. 21 is a time chart showing a relationship among a presentation position of a target controlled along the flowchart of FIG. 20, objective measurement operation, and an end determination flag.

### DETAILED DESCRIPTION

With respect to the use of plural and/or singular terms herein, those having skill in the art can translate from the plural to the singular and/or from the singular to the plural as is appropriate to the context and/or application. The various singular/plural permutations may be expressly set forth herein for sake of clarity.

An embodiment of an ophthalmologic apparatus according to the present disclosure will be described based on a first embodiment illustrated in the figures.

### First Embodiment

An ophthalmologic apparatus 1 of the first embodiment is an ophthalmologic apparatus of a both eyes open type capable of simultaneously measuring eye characteristics of both eyes when a patient opens his or her both eyes. In the ophthalmologic apparatus 1 of the first embodiment, an eye characteristic may be measured one eye by one eye by shielding one eye, or turning off a fixed target.

The ophthalmologic apparatus 1 of the first embodiment includes a support base 10, a measurement unit 20, an examiner's controller 30, a control portion 40, and a patient's controller (not illustrated). As seen from a patient, a left and right direction is an X direction, an up and down direction (vertical direction) is a Y direction, and a direction orthogonal to the X, Y directions (depth direction) is a Z direction.

The support base 10 includes a table 11 for optometry disposed on a floor and a column 12 rising from the table 11. The table 11 is a base on which a device and a tool for use in optometry such as an examiner's controller 30 are placed, and also a base for supporting a posture of a patient. The position (height position) of the table 11 in the Y direction may be adjustable.

The measurement unit 20 includes an arm 21, a measurement head 22, and a forehead rest 23. One end of the arm 21 is supported by the column 12 and the other end of the arm 21 extends toward a patient from the column 12 along the Z direction. The measurement head 22 is attached to the leading end portion of the arm 21. The measurement head 22 is thereby hung on the column 12 through the arm 21 above the table 11. The arm 21 is movable in the Y direction relative to the column 12. The arm 21 may be movable in the X, Z directions relative to the column 12.

The measurement head 22 measures an eye characteristic of a subject eye E. The measurement head 22 includes a driving portion 22a, and a pair of a left measurement portion 22L and a right measurement portion 22R that are provided under the driving portion 22a. The left measurement portion 22L and the right measurement portion 22R correspond to the left and right eyes of the patient, respectively. A left measurement optical system 25L that measures the eye characteristic of the left subject eye E of the patient is built in the left measurement portion 22L. A right measurement optical system 25R that measures the eye characteristic of the right subject eye E is built in the right measurement portion 22R. The measurement result by the measurement head 22 is input to the control portion 40.

The driving portion 22a moves each of the left measurement portion 22L and the right measurement portion 22R horizontally (X direction and/ Z direction) and vertically (Y direction), and rotates each of the left measurement portion 22L and the right measurement portion 22R in the X direction (left and right direction) about the rotation axis (Y axis) extending in the vertical direction and rotates each of the left measurement portion 22L and the right measurement portion 22R in the Y direction (up and down direction) about the rotation axis (X axis) extending in the horizontal direction.

The driving portion 22a rotates the left measurement portion 22L and the right measurement portion 22R in the X direction to converge or diverge the left and right subject eyes E. When the rotation axes that are the rotation centers of the left measurement portion 22L and the right measurement portion 22R position at the rotation point of the subject eye E (pass through rotation point of subject eye E), the driving portion 22a converges or diverges the left and right subject eyes E by rotating the left measurement portion 22L and the right measurement portion 22R in the X direction. On the other hand, to converge or diverge the left and right subject eyes when the rotation axes do not position at the rotation point of the subject eye E (does not pass through rotation point of subject eye E), it is necessary for the driving portion 22a to rotate the left measurement portion 22L and the right measurement portion 22R in the X direction after the rotation axis is aligned with the rotation point of the subject eye E horizontally moving the left measurement portion 22L and the right measurement portion 22R in the X direction and/or the Z direction.

The ophthalmologic apparatus 1 of the first embodiment objectively and subjectively measure an eye characteristic of the subject eye E. That is, an examiner performs voluntary objective examination and a subjective examination with the ophthalmologic apparatus 1. In the objective examination, the subject eye E is irradiated with light, and the information (eye characteristic) on the subject eye E is measured based on the detection result of the return light. The objective examination includes the measurement for obtaining the eye characteristic of the subject eye E and the photographing for obtaining the image of the subject eye E. The objective examination includes objective refraction measurement (refraction measurement), corneal shape measurement (kerato measurement), intraocular pressure measurement, ocular fundus photographing, tomogram photographing using optical coherence tomography (OCT) (OCT photographing), and measurement using OCT. In the subjective examination, a target is presented to a patient, and information (eye characteristic) on the subject eye E is measured based on response of a patient to the presented target. The subjective examination includes far examination, intermediate examination, near examination, contrast examination, subjective refraction measurement such as glare examination, and visual field examination.

Accordingly, the left measurement optical system 25L and the right measurement optical system 25R built in the measurement head 22 include an anterior eye segment observation system 150 that observes an anterior eye segment of the subject eye E and a target projection system 140 that presents a target to the subject eye E, as illustrated in FIG. 2. The left measurement optical system 25L and the right measurement optical system 25R include a refraction measurement projection system 160, a refraction measurement light receiving system 170, and a kerato measurement system 130 as the objective measurement optical system that objectively measures the eye characteristic of the subject eye E. The detailed configuration of the left measurement optical system 25L and the right measurement optical system 25R will be described later.

The forehead rest 23 is provided in the measurement unit 20, and is disposed between the left measurement portion 22L and the right measurement portion 22R. Apart (forehead) of a face of a patient contacts the forehead rest 23 during the measurement of the eye characteristic, so that the forehead rest 23 supports the face of the patient. That is, the patient facing the table 11 presses his or her forehead against the forehead rest 23 to stabilize the direction and the position of the face. The position of the forehead rest 23 is adjusted by moving the arm 21 in the Y direction relative to the column 12.

The examiner's controller 30 is an information processing device that outputs a control signal to the control portion 40 upon receipt of operation by an examiner. The examiner's controller 30 is a tablet terminal or a smart phone, for example, and is separated from the measurement unit 20 and is portable by an examiner. The examiner's controller 30 may be a laptop personal computer, a desk top personal computer, or a controller dedicated to the ophthalmologic apparatus 1. The examiner's controller 30 exchanges information with the control portion 40 via wireless communication and network communication.

As illustrated in FIG. 1, the examiner's controller 30 includes a display portion 31, an operation control portion (not illustrated), and an input button (not illustrated). The display portion 31 is a touch panel display provided on the surface of the examiner's controller, and an input button is set to the display portion 31. The operation control portion is a microcomputer built in the examiner's controller 30. The operation control portion controls an image that is displayed on the display portion 31 based on the measurement result and the detection result sent from the control portion 40. The operation control portion outputs the control signal according to the operation to the input button to the control portion 40.

The control portion 40 is an information processing device provided under the table 11. The control portion 40 totally controls each portion of the measurement unit 20 including the left measurement optical system 25L and the right measurement optical system 25R having the objective measurement optical system (refraction measurement projection system 160 and refraction measurement light receiving system 170, and kerato measurement system 130) and the target projection system 140. The control portion 40 sends the measurement result of the eye characteristic of the subject eye E measured by the measurement head 22 to the examiner's controller 30.

The control portion 40 presents (stimulates) a target to the subject eye E (refer to FIG. 2) with the left measurement optical system 25L and the right measurement optical system 25R, and changes the eye characteristic of the subject eye E (condition of subject eye E). The control portion 40 objectively measures the eye characteristic of the subject eye E when the target is presented to the subject eye E in time series. The control portion 40 feedbacks the result of the objective measurement of the eye characteristic to the operation control of each portion, and controls the operation of each portion of the ophthalmologic apparatus 1 after the target is presented to the subject eye (E) (after being stimulated). One example of the control to be performed by the control portion 40 will be described later.

The detailed configurations of the left measurement optical system 25L and the right measurement optical system 25R will be hereinafter described based on FIG. 2. As the left measurement optical system 25L and the right measurement optical system 25R have the same configuration, the description of the right measurement optical system 25 will be omitted, and the left measurement optical system 25L will be hereinafter only described. In the following description, "ocular fundus conjugate position P" is meant to be a position optically and substantially conjugate with an ocular fundus Ef of the subject eye E in an aligned state, and a position optically conjugate with the ocular fundus Ef of the subject eye E or a position near that position. "Pupil conjugate position Q" is meant to be a position optically and substantially conjugate with a pupil of the subject E in an aligned state, and a position optically conjugate with the pupil of the subject eye E or a position near that position.

The left measurement optical system 25L includes a Z alignment system 110, an XY alignment system 120, a kerato measurement system 130, a target projection system 140, an anterior eye segment observation system 150, a refraction measurement projection system 160, and a refraction measurement light receiving system 170, as illustrated in FIG. 2.

The anterior eye segment observation system 150 photographs a moving image of the anterior eye segment of the subject eye E. The imaging face of the imaging element 159 is disposed in the pupil conjugate position Q in the optical system via the anterior eye segment observation system 150. An anterior eye segment illumination light source 151 irradiates illumination light (e.g., infrared light) to the anterior eye segment of the subject eye E. The light reflected by the anterior eye segment of the subject eye E passes through the objective lens 152, transmits through a first dichroic mirror 153, transmits through a half mirror 154, passes through a first relay lens 155, a second relay lens 156, and transmits through a second dichroic mirror 157. The light transmitted through the second dichroic mirror 157 is imaged on the imaging face of the imaging element 159 (area sensor) by the first imaging lens 158. The imaging element 159 images and outputs a signal at a predetermined rate. The output (image signal) of the imaging element 159 is input to the control portion 40. The control portion 40 displays the anterior eye segment image E' based on the input image signal on the display portion 31 of the examiner's controller 30. The anterior eye segment image E' is an infrared moving image, for example.

The Z alignment system 110 projects light (infrared light) for the alignment in the optical axis direction (front and back direction, Z direction) of the anterior eye segment observation system 150 to the subject eye E. The light output from the Z alignment light source 111 is projected to the cornea of the subject eye E, is reflected by the cornea, and is imaged on the sensor surface of the line sensor 113 by the second imaging lens 112. When the position of the cornea apex is changed in the optical axis direction of the anterior eye segment observation system 150, the projection position of the light on the sensor surface of the line sensor 113 is changed. The control portion 40 obtains the position of the cornea apex of the subject eye E based on the projection position of the light on the sensor surface of the line sensor 113, and performs the Z alignment by controlling the driving portion 22a based on the obtained position.

The XY alignment system 120 irradiates light (infrared light) for the alignment in the direction (left and right direction (X direction), up and down direction (Y direction)) orthogonal to the optical axis of the anterior eye segment observation system 150 to the subject eye E. The XY alignment system 120 includes an XY alignment light source 121 provided in an optical path branched from the anterior eye segment observation system 150 by the half mirror 154. The light output from the XY alignment light source 121 is reflected by the half mirror 154, and is projected to the subject eye E through the anterior eye segment observation system 150. The reflection light by the cornea of the subject eye E is guided to the imaging element 159 through the anterior eye segment observation system 150.

The image (bright spot image) based on the reflection light by the cornea of the subject eye E is included in the anterior eye segment image E'. The control portion 40 displays the anterior eye portion image E' including the bright spot image and the alignment mark on the display portion 31. When the XY alignment is manually performed, a user performs a movement operation of the optical system to guide the bright spot image in an alignment mark. When the alignment is automatically performed, the control portion 40 controls the driving portion 22a to cancel the displacement of the bight point image to the alignment mark.

The kerato measurement system 130 projects, to a cornea, ring light flux (infrared light) for measuring the shape of the cornea of the subject eye E. The kerato plate 131 is disposed between the objective lens 152 and the subject eye E. Akerato ring light source (not illustrated) is provided rear (objective lens 152 side) of the kerato plate 131. The kerato measurement system 130 projects ring light flux to a cornea of the subject eye E by illuminating the kerato plate 131 with light from the kerato ring light source. The reflection light (kerato ring image) from the cornea of the subject eye E is detected together with the anterior eye portion image E' by the imaging element 159. The control portion 40 performs known calculation based on the kerato ring image to calculate the cornea shape parameter showing the shape of the cornea.

The target projection system 140 presents various targets such as a fixed target and a subjective examination target to the subject eye E. The light (visual light) output from the light source 141 is changed into parallel light flux by the collimate lens 142, and is irradiated to the eye chart 143. The eye chart 143 includes a transmission liquid crystal panel, and displays a target image Sf showing a target. The light transmitted through the eye chart 143 passes through the third relay lens 144, the fourth relay lens 145, is reflected on the first reflection mirror 146, transmits through the third dichroic mirror 168, and is reflected on the first dichroic mirror 153. The light reflected on the first dichroic mirror 153 is projected onto the ocular fundus Ef after passing through the objective lens 152. The light source 141, the collimate lens 142 and the eye chart 143 configure the target unit 147, and integrally movable in the optical axis direction.

When the subjective examination is performed, the control portion 40 moves the target unit 147 in the optical axis direction based on the result of the objective measurement. The control portion 40 controls the display of the eye chart 143 and displays the eye chart 143 selected by the examiner or the control portion 40 on the eye chart 143. Thereby, the target selected by the examiner or the control portion 40 is presented to a patient at a predetermined presentation position Pp. The control portion 40 receives the input of the response of the patient to the target and performs further control and calculation of a subjective test value. For example, in visual acuity measurement, the control portion 40 selects a next target based on the response to the Landolt ring, etc., to present the selected target, and determines the visual acuity value by repeating this process.

The target image Sf showing the target displayed by the eye chart 143 is not limited to those as long as it is used in optometry, and for example, a Landolt ring, a Snellen target, and an E chart are suitable. The target image Sf may be a still image or a moving image. Since the eye chart 143 includes a liquid crystal panel, it is possible to display the target image Sf in a desired shape, form, and contrast, allowing for objective and detailed optometry. The ophthalmologic apparatus 1 of the first embodiment is also equipped with two target units 147 corresponding to the left and right subject eyes E, respectively. Therefore, the ophthalmologic apparatus 1 can display the target that gives parallax corresponding to the predetermined presentation position Pp, and stereoscopic examination can be easily and precisely performed with a natural visual axis orientation.

The refraction measurement projection system 160 and the refraction measurement light receiving system 170 are objective optical systems for use in objective refraction measurement. The refraction measurement projection system 160 projects ring light flux (infrared light) for objective measurement onto the ocular fundus Ef. The refraction measurement light receiving system 170 receives the return light from the subject eye E of the ring light flux.

The refraction measurement light source 161 may be a superluminescent diode (SLD) light source, that is a high intensity light source with an emission diameter less than a predetermined size. The refraction measurement light source 161 is movable in the optical axis direction and is disposed at the ocular fundus conjugate position P. The ring diaphragm 165 (specifically, transmission portion) is disposed at the pupil conjugate position Q. The focusing lens 174 is movable in the optical axis direction. The focusing lens 174 may be a known variable focusing lens that can change its focal position under the control of the control portion 40. In the optical system via the refraction measurement light receiving system 170, the imaging surface of the imaging element 159 is disposed at the ocular fundus conjugate position P.

The light output from the refraction measurement light source 161 passes through the fifth relay lens 162 and enters the cone surface 163a (see FIG. 3B) of the cone prism 163. The light incident on the cone surface 163a is deflected and emitted from the bottom surface 163b (see FIG. 3B) of the cone prism 163. The light emitted from the bottom surface 163b of the cone prism 163 passes through the field lens 164 and through the transmission portion formed in a ring shape on the ring diaphragm 165. The light passing through the transmission portion of the ring diaphragm 165 (ring light flux) is reflected by the reflection surface of the holed prism 166, passes through the rotary prism 167, and is reflected by the third dichroic mirror 168. The light reflected by the third dichroic mirror 168 is reflected by the first dichroic mirror 153, passes through the objective lens 152, and is projected onto the subject eye E. The rotary prism 167 is used to average the light volume distribution of the ring light beam to the blood vessels and disease sites in the ocular fundus Ef and to reduce speckle noise caused by the light source.

The cone prism 163 is preferably disposed as close as possible to the pupil conjugate position Q.

The field lens 164 may have a ring diaphragm 165 affixed to the lens surface on the side of the subject eye E, for example, as shown in FIG. 3A. In this case, for example, a light shielding film is deposited on the lens surface of the field lens 164 so that a ring transmission portion is formed.

The refraction measurement projection system 160 may have a configuration in which the field lens 164 is omitted.

Furthermore, the cone prism 163 may have a ring diaphragm 165 affixed to the bottom surface 163b that emits light incident on the cone surface 163a after passing through the fifth relay lens 162, as shown in FIG. 3B, for example. In this case, for example, a light shielding film is deposited on the bottom surface 163b of the cone prism 163 so that a ring transmission portion is formed. The ring diaphragm 165 may be on the side of the cone surface 163a of the cone prism 163.

The ring diaphragm 165 may be a diaphragm with a transmission portion having a shape corresponding to a predetermined measurement pattern. The ring diaphragm 165 may have a transmission portion formed at a position eccentric to the optical axis of the refraction measurement projection system 160. The ring diaphragm 165 may have two or more transmission portions formed.

The return light of the ring light flux projected onto the ocular fundus Ef passes through the objective lens 152 and is reflected by the first dichroic mirror 153 and the third dichroic mirror 168. The return light reflected by the third dichroic mirror 168 passes through the rotary prism 167, through the hole in the holed prism 166, and through the sixth relay lens 171. The return light passing through the sixth relay lens 171 is reflected by the second reflection mirror 172 and passes through the seventh relay lens 173 and the focusing lens 174. The return light passing through the focusing lens 174 is reflected by the third reflection mirror 175, reflected by the second dichroic mirror 157, and formed on the imaging surface of the imaging element 159 by the first imaging lens 158. The control portion 40 calculates the refractive power value of the subject eye E by performing known calculation based on the output from the imaging element 159. For example, the refractive power value includes spherical power, astigmatism power and astigmatic axis angle.

A diaphragm (not shown) is placed between the holed prism 166 and the sixth relay lens 171 to limit the diameter of the light flux on the pupil. The transmission portion of this diaphragm is disposed at the pupil conjugate position.

Based on the calculated refractive power value, the control portion 40 moves the refraction measurement light source 161 and the focusing lens 174 in the optical axis direction so that the ocular fundus Ef, the refraction measurement light source 161, and the imaging surface of the imaging element 159 are optically conjugate. Furthermore, the control portion 40 moves the target unit 147 in the direction of its optical axis in conjunction with the movement of the refraction measurement light source 161 and the focusing lens 174. The target unit 147 including the light source 141, collimate lens 142 and eye chart 143, the refraction measurement light source 161, and the focusing lens 174 may be movable in conjunction and in the direction of their respective optical axes.

An example of the control performed by the control portion 40 of the first embodiment will be described below based on the flowchart shown in FIG. 4. In the flowchart of FIG. 4, the control portion 40 presents a target to the left and right subject eyes E, respectively, and performs objective refraction measurement in the binocular vision state in time series manner, and then determines to terminate the presentation of the target based on the result of objective measurement. The following is a description of a case in which the control portion 40 terminates the presentation of the target according to the determination result and also determines the eye characteristics.

In Step S1, the control portion 40 controls the eyechart projection system 140 to present the target at the predetermined presentation position Pp (see FIG. 5) that is set in advance, and proceeds to Step S2. At this time, the patient sees the target presented at the presentation position Pp with the left and right subject eyes E, respectively. Note that "presentation position Pp" indicates the optical position where the target is presented. The predetermined presentation position Pp set in Step S1 is, for example, set to a position where the objective measurement of the subject eye E is performed beforehand (however, subject eye E may be adjusted in this state) and the power of the subject eye E obtained at that time (spherical power, astigmatism power, etc.) is cancelled. The visual line direction at this time is set to infinity where the visual line directions of the left and right subject eyes E are parallel. In this case, the subject eye E is corrected and set to zero D (diopter).

In step S2, following the presentation of the target in Step S1, the control portion 40 changes the presentation position Pp of the target to a far presentation position Ppx (see FIG. 5) that is farther from the far point of the subject eye E. Here, the far presentation position Ppx can be any position farther than the presentation position Pp (position set in direction farther from far point), for example, +2D based on the presentation position Pp (zero D) set in step S1. The far presentation position Ppx may be set at any position within +1D to +3D based on the presentation position Pp (zero D) set in step S1, for example, or it may be set at an infinity position (Pp0).

After the far presentation position Ppx is set, the control portion 40 moves the target unit 147 of the target projection system 140 along its optical axis to present the target at the far presentation position Ppx, and proceeds to Step S3. After a predetermined time (e.g., 3 seconds) has elapsed after the control portion 40 presents the target at the far presentation position Ppx, proceeds to Step S3. This allows the control portion 40 to present the target at the far presentation position Ppx by the eye chart projection system 140 for a predetermined time. In addition, this allows the patient to continuously view the eye target presented at the far presentation position Ppx for the predetermined time.

In Step S3, following the presentation of the target at the far presentation position Ppx in Step S2, the control portion 40 objectively measures the eye characteristics of the left and right subject eyes E respectively, and proceeds to Step S4. Here, the objective measurement of the subject eye E is an objective refraction measurement (refraction measurement) performed using an objective measurement optical system (e.g., refraction measurement projection system 160 and refraction measurement light receiving system 170). When the objective examination is performed in Step S3, the control portion 40 continues to have the target continuously presented at the far presentation position Ppx. On the other hand, the examination eye E is clouded because it views the target presented at the far presentation position Ppx that is farther from the far point. Therefore, the control portion 40 measures the eye characteristics of the subject eye E in the clouded state.

Furthermore, the result of the objective measurement of the subject eye E is shown in a graph with time on the horizontal axis and the objective refraction measurement value on the vertical axis, as shown in FIG. 6, for example. Here, the more positive the objective refraction measurement value becomes (upper side in FIG. 6), the more it means that the adjustment of the subject eye E has been removed.

In step S4, following the implementation of the objective measurement in Step S3, the control portion 40 determines whether or not the flag for determining the end of the presentation of the target has been turned on based on the measurement result from the objective measurement. If the control portion 40 determines YES (end determination flag = ON), proceeds to Step S5, and determines NO (end determination flag = OFF), the process returns to Step S3. In other words, the control portion 40 repeatedly executes the objective measurement of the eye characteristics of the subject eye E until the end determination flag turns on.

The flag for determining the end of the presentation of the target is turned on when any of the conditions 1 to 3 listed below are satisfied, for example.
(Condition 1) As shown in FIG. 7A, when the measured value has not changed since the initial measurement at the time (time n) when the predetermined time has elapsed since the presentation of the target at the far presentation position Ppx (time t0).
(Condition 2) As shown in FIG. 7B, when the measured values at the time (time m+n) when the predetermined time has elapsed since the time when the target is presented at the far presentation position Ppx (time t0) have not changed compared to the value measured at the predetermined time (time m) before that.
(Condition 3) When the rate of change of the measured value reaches an inflection point. That is, when the second derivative of the graph shown in FIG. 6 becomes a negative value.

The measurement value x at the time when a predetermined time (e.g., 1 minute) has elapsed (time t shown in FIG. 6) since the time when the measurement of the eye characteristic starts (time t0) can be estimated based on the rate of change of the measurement value during the predetermined measurement time (e.g., 3 seconds: time until time t1 shown in FIG. 6) from the time when measurement starts (time t0) Therefore, the control portion 40 can estimate the timing when the measurement value saturates (change in measurement value converges) based on the rate of change of the measurement value during the period from immediately after the start of measurement (time t0) to the predetermined measurement time (time 11). Therefore, for example, the flag for determining the end of presentation of the target may be turned on when the timing at which the measurement value estimated by the control portion 40 becomes saturated (convergence of changes) has elapsed after the target is presented at the far presentation position Ppx. The control portion 40 may also inform a user of the timing at which the estimated measurement value saturates.

Furthermore, the objective measurement of the eye characteristic of the subject eye E is repeatedly performed until the flag for determining end is turned on. Therefore, the control portion 40 objectively measures the eye characteristic in time series when the subject eye E views the target. Here, "time-series objective measurement" is meant that the objective measurement of the eye characteristic is performed several times (two or more) by the control portion 40 during a certain period of time, and temporal change in the measurement result is detected.

In step S5, following the determination that the end determination flag = ON in step S4, the control portion 40 terminates the presentation of the target and also determines the effect of the target visual recognition at the end of the target presentation, and proceeds to the end. In other words, the control portion 40 controls the operation of each part of the ophthalmologic apparatus 1 based on the determination that the end determination flag = ON in Step S4.

Here, the determination of the effect of the visual recognition of the target is made according to the reason why the end determination flag is turned on (difference in satisfaction condition), as listed below.

When the end determination flag is turned on because the first condition is satisfied, the determination is made that "there is no change in eye characteristic even if the target is viewed (no cloud and fog effect due to viewing of target)".

When the second condition is satisfied and the end judgment flag is turned on, the judgment is made that "there was a certain cloud and fog effect (change in ocular characteristics) due to viewing of the target, but there is no change in ocular characteristics (no cloud and fog effect) even if viewing of the target is continued (after a predetermined time (time m+n) has passed)".

When the third condition is satisfied and the end judgment flag is turned on, the judgment is made that "a certain cloud and fog effect (change in ocular characteristics) caused by viewing the target has occurred, but the time when the maximum cloud and fog effect (the greatest change in ocular characteristics) is produced by viewing the target has passed.

Furthermore, the control portion 40 displays the result of the determination of the effect by the visual recognition of the target on the display portion 31 of the examiner's controller 30. When the presentation of the target is terminated in Step S5, the control portion 40 may inform (announce) that the presentation of the target ends. The control portion 40 may inform the end of the presentation by displaying on the display portion 31 of the examiner's controller 30 or by voice.

The effect of the ophthalmologic apparatus 1 in the first embodiment is described as follows.

The ophthalmologic apparatus 1 of the first embodiment presents a target to the left and right subject eyes and objectively measures the eye characteristic of the subject eye E with the subject eye E visually recognized the target. The ophthalmologic apparatus 1 then determines whether or not to terminate the presentation of the target based on the results of the objective measurement, and repeatedly measures the eye characteristic until the predetermined condition is met. When the predetermined condition is satisfied, the ophthalmologic apparatus 1 terminates the presentation of the target and determines the effect of the visual recognition of the target.

In other words, the ophthalmologic apparatus 1 of the first embodiment objectively measures the eye characteristic in time series after stimulating the subject eye E by presenting the target. In other words, the ophthalmologic apparatus 1 of the first embodiment quantitatively monitors the adjustment state of the patient. The ophthalmologic apparatus 1 controls the operation of each portion of the ophthalmologic apparatus 1 based on the measurement result of the objective measurement. Here, "controlling operation of each portion" is meant to, in the first embodiment, terminate the presentation of the target and determine the effect of the visual recognition of the target. In addition, "controlling operation of each portion" may include reporting the termination of the presentation of the target.

As a result, the ophthalmologic apparatus 1 of the first embodiment can automatically perform the termination of the presentation of the target based on the measurement result of the eye characteristic of the subject eye E objectively measured in time series. In other words, the ophthalmologic apparatus 1 of the first embodiment can automatically control the operation of the target projection system 140 of the ophthalmologic apparatus 1 according to the condition (eye characteristic) of the subject E after being stimulated as "visually recognizing target presented at predetermined position". As a result, the ophthalmologic apparatus 1 of the first embodiment can feedback the effect of the target visual recognition to the operation control of the target projection system 140. In addition, by automatically controlling the operation of the target projection system 140, the ophthalmologic apparatus 1 can suppress variation in the condition of the subject eye E when the target presentation is terminated, because the examiner or other person's subjectivity is not involved when controlling the operation of "termination of target presentation". The ophthalmologic apparatus 1 of the first embodiment can appropriately control the operation of the target projection system 140 based on objective information (measurement result of subject).

The ophthalmologic apparatus 1 of the first embodiment is also capable of analyzing the effect of presenting the target based on the measurement result of the eye characteristic (data on change in adjustment state during repeated cloud operation) and objectively indicating the effect of target visual recognition (here, the cloud effect) by the objective measurement. The ophthalmologic apparatus 1 can then provide a reason for the effect of the presentation of the target (target visual recognition), in other words, it can provide an objective rationale for the effect of target visual recognition.

In the ophthalmologic apparatus 1 of the first embodiment, the far presentation position Ppx is set at a position farther from the far point of the subject eye E. The control portion 40 continues to have the target continuously presented at the far presentation position Ppx during the objective measurement of the eye characteristic of the subject eye E. In other words, the control portion 40 causes the objective measurement optical system (reflection measurement projection system 160 and reflection measurement light receiving system 170) to perform the objective measurement when the patient visually recognizes the target presented at the far presentation position Ppx and is made to be in a clouded state.

As a result, the ophthalmologic apparatus 1 of the first embodiment can perform objective measurement of a change in the eye characteristic (adjustment state) of the subject eye E during the cloud fogging operation. Therefore, the ophthalmologic apparatus 1 of the first embodiment can objectively determine whether or not the effect of cloud fogging has been produced by presenting a target at the far presentation position Ppx, when the effect of cloud fogging has been produced, how long the effect of cloud fogging will continue, and the like. Furthermore, the ophthalmologic apparatus 1 of the first embodiment can reflect a change in the adjustment state when the subject eye E is clouded based on the result of such determination in the operation control of the ophthalmologic apparatus 1.

### Second Embodiment

An ophthalmologic apparatus 1A in the second embodiment has the same configuration as the ophthalmologic apparatus 1 in the first embodiment. On the other hand, the control portion 40 of the second embodiment changes the presentation position Pp continuously or gradually when presenting a target to the subject eye E before objectively measuring the eye characteristic.

An example of the control performed by the control portion 40 of the second embodiment is described below based on the flowchart shown in FIG. 8. Since the basic operation and control of each step is the same as in the first embodiment, the detailed description will be omitted.

In Step S11, the control portion 40 sets the presentation position Pp of the target to a first presentation position Pp1 near the far point of the subject eye E (e.g., position shifted within ± 1D with respect to presentation position Pp (zero D) set in step S1 in first embodiment) as shown in FIG 9. The control portion 40 then moves the target unit 147 of the target projection system 140 along its optical axis to present the target at the first presentation position Pp1, and proceeds to Step S12. The control portion 40 may proceed to step S12 after a predetermined time has elapsed after presenting the target at the first presentation position Pp1. In this case, the control portion 40 can make the target presented at the first presentation position Pp1 continuously visible to the subject eye E for a predetermined time.

In Step S12, following the presentation of the target at the first presentation position Pp1 in Step S11, the control portion 40 changes the presentation position Pp of the target to a second presentation position Pp2 at a position farther (+ side) than the first presentation position Pp1 (e.g., based on presentation position Pp (zero D) set in Step S1 in the first embodiment + (any position within +1D to +3D). The control portion 40 then presents the target at the second presentation position Pp2, and proceeds to Step S13. The control portion 40 proceeds to Step S13 after a predetermined time has elapsed after presenting the target at the second presentation position Pp2. In this way, the control portion 40 can make the target presented at the second presentation position Pp2 continuously visible to the subject eye E for a predetermined time. The second presentation position Pp2 may be set at an infinity position (Pp0) where the viewing directions of the left and right subject eyes E are parallel.

When the presentation position Pp of the target is changed from the first presentation position Pp1 to the second presentation position Pp2, the control portion 40 changes the presentation position Pp continuously or gradually. When the presentation position Pp is changed continuously, the control portion 40 changes the presentation position Pp gradually with the target being presented. In the case of the gradual change of the presentation position Pp, the control portion 40 temporarily interrupts the presentation of the target and switches the presentation position Pp.

Furthermore, as shown in FIG. 9, the control portion 40 causes the target projection system 140 to present a different target (target image Sf) according to the presentation position Pp when the target is presented to the target projection system 140. In other words, the control portion 40 of the second embodiment controls the target projection system 140 so that the size of the target image Sf changes on the eye chart 143 as the presentation position Pp of the target changes. Here, the target image Sf2 presented at the second presentation position Pp2 is smaller than the target image Sf1 presented at the first presentation position Pp1, which is closer to the second presentation position Pp2. The target image Sf0 presented at the infinity position (Pp0) is presented even smaller than the target image Sf2 presented at the second presentation position Pp2.

In the ophthalmologic apparatus 1A of the second embodiment, the control portion 40 presents the same target to each of the left and right subject eyes E, and fuses. Then, the control portion 40 changes the distance Dp from the left and right subject eyes E to the fused target (presentation position Pp) when the target is presented to the target projection system 140. In other words, the control portion 40 increases the distance Dp1 to the fused image target presented at the first presentation position Pp1 to the distance Dp2 by presenting the target at the second presentation position Pp2. The control portion 40 controls a driving portion 22a so that the target presented at the distance Dp2 (second presentation position Pp2) is visually recognized and in focus with both eyes, and controls the rotation angle θ by rotating the left measurement porting 22L and the right measurement portion 22R in the X direction. In this case where the distance Dp is longer, the rotation angle θ becomes smaller, and the left and right subject eyes open and diverge according to the change in distance Dp. "Rotation angle θ" is the angle of the optical axis of the target projection system 140 (direction of optical axis from subject eye E to fused image target) with respect to the state in which the target is presented at infinity (parallel to each other).

In Step S13, following the presentation of the target at the second presentation position Pp2 in Step S12, the control portion 40 objectively measures the eye characteristic of the left and right subject eyes E respectively, and proceeds to Step S14.

In Step S14, following the implementation of the objective measurement in Step S13, the control portion 40 determines, based on the measurement results from the objective measurement, whether or not the flag for determining the end of the target presentation has turned on. If the control portion 40 determines YES (end determination flag = ON), it proceeds to Step S15, and if NO (end determination flag = OFF), it returns to Step S11.

In the second embodiment, in addition to the cases where any of the conditions 1 to 3 shown in the first embodiment are satisfied, the end determination flag of the target presentation is also turned on when the following condition 4 is satisfied. (Condition 4) When the final objective measurement of the subject eye E changes to the positive side of the target presentation position Pp from before it is repeated between the first presentation position Pp1 and the second presentation position Pp2.

If the end determination flag is not turned on, the control portion 40 returns the presentation position Pp of the target from the second presentation position Pp2 to the first presentation position Pp1 near the far point, and presents the target at the first presentation position Pp1 again. At this time, the distance Dp2 to the fused image target presented at the second presentation position Pp2 is shortened to the distance Dp1 by returning the presentation position Pp to the first presentation position Pp1. The control portion 40 controls the driving portion 22a so that the target presented at the distance Dp1 (first presentation position Pp1) is visually recognized and in focus with both eyes, and controls the rotation angle θ by rotating the left and right measurement portions 22L and 22R in the X direction. In this case where the distance Dp becomes shorter, the rotation angle θ becomes larger, and the left and right subject eyes E converge according to the change in the distance Dp.

Then, the control portion 40 causes the target to be presented at the second presentation position Pp2 again after a predetermined time has elapsed since the target is presented at the first presentation position Pp1. In this way, the ophthalmologic apparatus 1A of the second embodiment controls the target projection system 140 so that the presentation position Pp of the target changes repeatedly between the first presentation position Pp1 near the far point of the subject eye E and the second presentation position Pp2 farther than the first presentation position Pp1 until the end determination flag is turned on. In other words, the control portion 40 of the ophthalmologic apparatus 1A in the second embodiment controls the target projection system 140 so that the focus position of the target moves back and forth when the target is presented on the target projection system 140.

In Step S15, following the determination of the end determination flag = ON in Step S14, the control portion 40 terminates the presentation of the target and also determines the effect of the target visual recognition at the end of the target presentation, and proceeds to the end.

As described above, the ophthalmologic apparatus 1A in the second embodiment changes the presentation position Pp of the target from the first presentation position Pp1 to the second presentation position Pp2 continuously or gradually when the target is presented before the eye characteristic is objectively measured. In this way, the ophthalmologic apparatus 1A in the second embodiment can forcibly change the adjustment state of the subject eye E, enabling the subject eye E to undergo training of the ciliary body and other eye muscles. When the eye muscle is trained to improve the muscle strength of the subject eye E, it is possible to improve the lack of blood flow to the subject eye E and to eliminate the lack of oxygen. The ophthalmologic apparatus 1A in the second embodiment can then objectively determine the effect of the eye muscle training by objectively measuring the eye characteristic during the eye muscle training.

In particular, in the ophthalmologic apparatus 1A of the second embodiment, the control portion 40 repeatedly changes the presentation position Pp between the first presentation position Pp1 near the far point of the subject eye E and the second presentation position Pp2 farther than the first presentation position Pp1 when the target is presented. Thus, the ophthalmologic apparatus 1A in the second embodiment can relax the ciliary body and other ocular muscles of the subject eye E and make the patent perform cloud training. The ophthalmologic apparatus 1A of the second embodiment can objectively demonstrate the effect of the cloud training by the result of the objective measurement while performing the cloud training.

In addition, if the subjective examination is performed while the adjustment is intervening, the examination is performed in a state of overcorrection, which may result in the progression of myopia and eyestrain. In contrast, the ophthalmologic apparatus 1A in the second embodiment can objectively show the effect of cloud training, so the examiner and others can conduct the subjective examination based on an accurate understanding that the adjustment of the subject eye E has been removed. Therefore, the ophthalmologic apparatus 1A of the second embodiment can perform the subjective examination with the adjustment removed.

Furthermore, although pseudomyopia is eased in the subject eye E when the adjustment is removed, it is unclear whether the state in which pseudomyopia is eased is sustained. In contrast, the ophthalmologic apparatus 1A of the second embodiment can objectively demonstrate the effect of the cloud training by the result of the objective measurement. Therefore, the ophthalmologic apparatus 1A of the second embodiment can quantitatively determine the state in which pseudomyopia is eased (whether or not adjustment has been removed), and it is possible to estimate how long the state in which pseudomyopia is eased will last.

Furthermore, in the ophthalmologic apparatus 1A of the second embodiment, as shown in FIG. 9, the control portion 40 causes the target projection system 140 to present a different target (target image Sf) according to the presentation position Pp so that the farther the presentation position Pp is, the smaller the target is presented. This allows the ophthalmologic apparatus 1A of the second embodiment to make the patient recognize the target image Sf as being farther away simply by visually recognizing the target image Sf with the subject eye E.

The target image Sf may be displayed as a moving image, with continuous changes in size. In this case, the patient can feel more naturally that the object, etc. indicated in the target image Sf moves away. In addition, the target image Sf in the second embodiment should be one that has been seen approaching or moving away in the real world in order to make the recognition of the change in presentation position Pp more natural, and in the second embodiment, a car is used as an example. In the example shown in FIG. 9, the target image Sf is simply an image of a car, but it may also show a simple background (e.g., straight road) to easily recognize perspective. The target image Sf may be frame-by-frame display of multiple still images, as long as the size of the target image Sf can change so that the viewer can recognize that the presentation position Pp has changed. In addition, the manner in which the target image Sf changes, such as the magnification rate, and the content to be drawn may be set as needed, and is not limited to the configuration of the second embodiment. Furthermore, the same image (image whose size does not change) may be used for the target image Sf regardless of the presentation position Pp of the target.

The ophthalmologic apparatus 1A of the second embodiment presents the same target to the left and right examination eyes E, and fuses the images. The control portion 40 increases the distance Dp1 to the fused image target presented at the first presentation position Pp1 to the distance Dp2 by presenting the target at the second presentation position Pp2, thereby reducing the rotation angle θ and opening the left and right subject eyes E. The control portion 40 also shortens the distance Dp2 to the fused image target presented at the second presentation position Pp2 to the distance Dp1 by returning the presentation position Pp of the target from the second presentation position Pp2 to the first presentation position Pp1. In this way, the control portion 40 increases the rotation angle θ and converges the left and right subject eyes E.

In this way, the ophthalmologic apparatus 1A in the second embodiment can make the left and right subject eyes converge and open by presenting the target, and can make the ciliary body and other eye muscles of the subject eye E repeat tension and relaxation. This allows the ophthalmologic apparatus 1A of the second embodiment to perform eye muscle training on the subject eye E.

The ophthalmologic apparatus of the present disclosure has been described based on Examples 1 and 2. The specific configuration is not limited to these examples, and design changes and additions are permitted as long as they do not depart from the gist of the invention claimed in each claim.

In the ophthalmologic apparatus 1 of the first embodiment, after a target is presented at a predetermined presentation position Pp set at zero D based on the previously measured objective measurement value, a target is presented at a distant presentation position Ppx set at +2 D. Then, after a predetermined time (e.g., 3 seconds) has elapsed, an example of objective measurement of an eye characteristic was shown. However, the presentation position Pp of the target and the presentation time of the target can be set arbitrarily.

That is, for example, the control portion 40 sets "far presentation position Ppx" to a position +2D off the fully corrected value of the subject eye E and causes the target to be presented at the far presentation position Ppx set at +2D off the fully corrected value. As a result, the test eye E is in the same condition as when wearing a lens +2D from the fully corrected value. Furthermore, the control portion 40 makes the target presented at the distance presentation position Ppx visible to the subject eye E for at least 10 minutes (e.g., 15 minutes) and continues to cloud the test eye E for at least 10 minutes. Then, the control portion 40 objectively measures the eye characteristic of the test eye E after 10 minutes or more (e.g., 15 minutes) of continuous cloud fogging.

In this way, the ophthalmologic apparatus 1 of the first embodiment may relax the tension of the subject eye E by presenting the target and continuing the cloud fogging so that the state becomes the same as when a lens fully corrected to +2D is worn for 15 minutes (more than 10 minutes).

In the ophthalmologic apparatus 1A of the second embodiment, an example was shown in which the presentation position Pp repeatedly changes between the first presentation position Pp1 near the far point of the subject eye E and the second presentation position Pp2 farther than the first presentation position Pp1 when a target is presented by the target projection system 140. However, the control portion 40 may control the target projection system 140 so that the presentation position Pp of the target changes repeatedly between, for example, the first presentation position Pp1 near the far point of the subject eye E and the third presentation position Pp3 closer (- side) than the first presentation position Pp1 (see FIG. 9). In this case, the focus position of the target also moves back and forth.

When the presentation position Pp of the target changes repeatedly between the first presentation position Pp1 near the far point and the third presentation position Pp3 closer to that, the ophthalmologic apparatus can strain the ciliary body and other ocular muscles of the subject eye E, and make the subject eye E train its adjustment. The ophthalmologic apparatus can then objectively determine the effect of the adjustment training of the subject eye E by performing the objective measurement while performing the adjustment training.

Furthermore, even when the presentation position Pp of the target changes between the first presentation position Pp1 and the third presentation position Pp3, the control portion 40 may present a different target (target image Sf) according to the presentation position Pp. In this case, as shown in FIG. 9, the target image Sf3 presented at the third presentation position Pp3 is presented larger than the target image Sf1 presented at the first presentation position Pp1, which is farther from the third presentation position Pp3. This allows the ophthalmologic apparatus to make the patient recognize the target image Sf as approaching simply by visually recognizing the target image Sf with the subject eye E.

The presentation position Pp of the target does not only change from the vicinity of the far point (first presentation position Pp1) to the far position (second presentation position Pp2) or from the vicinity of the far point (first presentation position Pp1) to the near position (third presentation position Pp3). The presentation position Pp of the target may change from the position farther than the far point (second presentation position Pp2) to the vicinity of the far point (first presentation position Pp1) and even the position closer than the far point (third presentation position Pp3). Furthermore, the presentation position Pp may change from any position to any position. The presentation position Pp does not have to change before the measurement of the eye characteristic, and may be set at a predetermined position (e.g., second presentation position Pp2) from the beginning.

In the ophthalmologic apparatus 1 of the first embodiment, it is described that the target image Sf showing the target displayed by the eye chart 143 is not particularly limited as long as it is used for optometry, for example, a Landolt ring, a Snellen target, an E charts, and the like are suitable. In the second embodiment, it is also described that the target image Sf is preferably one that has been seen approaching or moving away in the real world, with an automobile as an example. However, the eye chart 143 of the target projection system 140 may present a background image Sb around the target at the same time as the target image Sf showing the target, as shown in FIG. 10A. In the example shown in FIG. 10A, a ship is shown as the target image Sf, and the sun, sea, and guardrail are presented as the background image Sb.

Furthermore, as shown in FIG. 10B, when the control portion 40 causes the target projection system 140 to present the target (target image Sf), it sets the presentation position Pp of the target between the near and far points of the subject E so that the target (target image Sf) is in focus of the subject E.
On the other hand, the background image Sb may be presented at a position farther from the far point of the subject E so that the background image Sb is not in focus (blurred). In other words, the control portion 40 may cause an image to be presented to the subject E in which only the target (target image Sf) is in focus and the background image Sb is blurred. This allows the ophthalmologic apparatus to make it easier for the subject eye E to see and fix on the target to be visually recognized. In this case, the control portion 40 objectively measures the eye characteristic of the subject eye E, which visually recognizes an image in which the background image Sb is blurred and the target image Sf is in focus. As a method of blurring the background image Sb, it is difficult to change the distance Dp to the target on the same image. Therefore, the background image Sb can be blurred by simulating the blurred portion by ray tracing or by applying some kind of filter only to the background image Sb. The method of blurring the background image Sb may be to create the target image Sf and the background image Sb in the eye chart 143 separately and display them so that they overlap by separating their optical paths, and to display the background image Sb by shifting its position in the front and back direction relative to the target image Sf.

When having the target projection system 140 present the target (target image Sf), the control portion 40 may alternately present the wide-angle image α shown in FIG. 11A and the narrow angle image β shown in FIG. 11B. Here, "wide angle image α" and "narrow angle image β" can be any image for which the angle of view of the captured image is "narrow angle image β < wide angle image α". The size of the angle of view can be set arbitrarily. The presentation position Pp of the target may be changed to any position (far point vicinity position, position farther away from far point, or position closer to far point) as the image is switched between the wide angle image α and the narrow angle image β. The presentation position Pp of the target may be fixed regardless of switching between the wide angle image α and the narrow angle image β. By having the control portion 40 alternately present the wide angle image α and the narrow angle image β to the target projection system 140, the ophthalmologic apparatus can cause the subject eye E to train the ciliary body and other eye muscles.

In addition, the control portion 40 may control the target projection system 140 to present the target (target image Sf) to each of the left and right subject eyes E, and as shown in FIG. 12, to give disparity between the target presented to the left subject eye E (left target image SfL) and the target presented to the right subject eye E (right target image SfR) according to the presentation position Pp. The left target image SfL and the right target image SfR shown in FIG. 12 are a pair of perspective images with disparity according to the presentation position Pp. In the example shown in FIG. 12, the gazing target T is presented overlaid on each of the left and right target images SfL and SfR. This allows the ophthalmologic apparatus to provide a stereoscopic view of the target and allows the subject eye E to train the ciliary body and other eye muscles.

Furthermore, the target image Sf may be a moving image of an airplane or other object moving at high speed in the eye chart 143. The control portion 40 also sets a plurality of target presentation areas A within the eye chart 143, as shown in FIG 13A. Then, the control portion 40 causes a visual mark (visual mark image Sf) to be presented in any one of the plurality of targe presentation areas A (see FIG. 13B), followed by a visual mark (visual mark image Sf) in one of the different target presentation areas A (see FIG. 13C). In other words, when presenting a target (target image Sf) by the target projection system 140, the control portion 40 may change the presentation position Pp of the target in the range where the target is presented (in eye chart 143) in the vertical or horizontal direction, or the vertical and horizontal direction as appropriate.

This causes the patient to move the gaze direction in accordance with the position where the target (target image Sf) is presented, which stretches and contracts the external eye muscle (superior rectus, inferior rectus, external rectus, internal rectus, superior oblique, and inferior oblique muscles) that move the eye, training the eye muscle. The control portion 40 may then make the patient look at the target that causes the external ocular muscle to stretch and contract, and then perform other objective measurements of eye characteristic.

In other words, the control portion 40 sets the presentation position Pp of the target to the differential presentation position Pp10 (see FIG. 13B, 13C) where the gaze direction is shifted in the up and down direction and/or the right and left direction relative to the front normal viewing state. Then, the control portion 40 causes the target projection system 140 to present the target at the difference presentation position Pp10, and when the subject eye E visually recognizes the target, the objective measurement is performed by the objective measurement optical system (refraction measurement projection system 160 and refraction measurement light receiving system 170).

This enables the ophthalmologic apparatus 1 to objectively measure the change in the eye characteristic (adjustment state) of the subject eye E when the external eye muscles are stretched or contracted. The ophthalmologic apparatus 1 can then objectively determine whether or not the eye muscle training has been effective, when the eye muscle training has been effective, how long the ocular muscle training will continue to be effective, etc. by presenting a target at the difference presentation position Pp10. Furthermore, in this case, the ophthalmologic apparatus 1 can reflect the changes in the adjustment state of the eye muscle of the subject eye E when they are trained in the operation control of the ophthalmologic apparatus 1 based on the results of said determination.

In addition, the control portion 40 may have the target images Sf of different brightness presented in sequence. In this case, the ciliary body is trained because the ciliary body stretches and contracts to adjust the amount of light entering the eye. In other words, the control portion 40 may have the patient visually recognize the targets that cause the ciliary body to stretch and contract before performing the objective measurement of the eye characteristic.

Furthermore, the control portion 40 may perform the objective measurement of the eye characteristic with the external eye muscles and ciliary body stretched and contracted by sequentially presenting target that vary in the front and back position, the up and down position, and the left and right position of the presentation position Pp of the target, as well as the brightness of the target in a complex manner.

In the ophthalmologic apparatus 1 of the first embodiment, an example is shown in which the operation of each portion of the ophthalmologic apparatus 1 controlled by the control portion 40 in response to the result of the objective measurement of the subject eye E is "end of the presentation of target and determination of the effect of target recognition". However, the content of the operation controlled by the control portion 40 can be changed arbitrarily. That is, the operation of the ophthalmologic apparatus 1 controlled by the control portion 40 may be, for example, reporting the end of the presentation of the target, changing the target to be presented, changing and setting the presentation position Pp of the target, and the like. Furthermore, the operation of the ophthalmologic apparatus 1 controlled by the control portion 40 may be, for example, the end of the objective measurement or the reporting of the end of the obj ective measurement.

Furthermore, the ophthalmologic apparatus 1 of the first embodiment, when determining the effect of the target presentation, may not only determine whether or not there is an effect, but may also indicate the amount of adjustment relaxation or show a trend of change in adjustment in cloud training. Specifically, the ophthalmologic apparatus 1 of the first embodiment may estimate and indicate whether or not it is worth having the patient perform cloud training, or how long the patient should be allowed to perform cloud training.

The ophthalmologic apparatus 1 of the first embodiment is shown as an example of conducting a binocular examination by presenting a target to the left and right subject eyes E, respectively. However, the ophthalmologic apparatus 1 of the first embodiment can also examine the subject eye E one eye at a time.

In the ophthalmologic apparatus 1A of the second embodiment, an example is shown in which objective measurement is performed after changing the presentation position Pp of the target. However, this is not limited to this, and the control portion 40 may also perform the objective measurement while the target is presented. That is, for example, after the control portion 40 has the target presented at the first presentation position Pp1, it starts the objective measurement of the eye characteristic of the subject eye E. Then, the control portion 40 continues to perform the objective measurement while changing the presentation position Pp of the target, for example, from the first presentation position Pp1 to the second presentation position Pp2, etc. Based on the objective measurement values after visually recognizing the second presentation position Pp2 or while changing the presentation position Pp, the control portion 40 determines the end of the presentation of the target, determines the effect of the target visual recognition, and measures the adjustment relaxation amount of the subject eye E.

By continuing to perform the objective measurement while the presentation position Pp of the target changes, the ophthalmologic apparatus of the present disclosure can objectively measure the eye characteristic of the subject eye E while looking at the target whose presentation position Pp changes, and can understand the change in eye characteristic that occurs as the presentation position Pp of the target changes.

An example of the specific control for performing the objective measurement during the presentation of the target is described below, based on the flowcharts shown in FIG. 14 or FIG. 16. Since the basic operation and control of each step are the same as in the first embodiment and the second embodiment, detailed explanations will be omitted.

In Step S21, the control portion 40 sets the presentation position Pp of the target to the first presentation position Pp1 near the far point of the subject eye E (see FIG. 9, for example, a position shifted within ± 1D with respect to presentation position Pp (zero D) set in Step S1 in first embodiment). The control portion 40 then moves the target unit 147 of the target projection system 140 along its optical axis to present the target at the first presentation position Pp1, and proceeds to Step S22. In the example shown in FIG. 14, the presentation position Pp of the target is set to the first presentation position Pp1 in Step S21, but the control portion 40 can set the presentation position Pp of the target to any position in Step S21.

In Step S22, following the presentation of the target at the first presentation position Pp1 in Step S21, the control portion 40 starts the objective measurement of the eye characteristic of the left and right subject eyes E, respectively, and proceeds to Step S23. The objective measurement of the eye characteristic is continued after Step S22.

In Step S23, following the start of the objective measurement in step S22, the control portion 40, while presenting the target, continuously changes the presentation position Pp of the target from the first presentation position Pp1 to the second presentation position Pp2 at a position farther (+ side) than the first presentation position Pp1 (e.g., any position within +1D to +3D with reference to presentation position Pp (zero D) set in Step S1 in first embodiment), and proceeds to step S24. Here, the control portion 40 continues to perform the objective measurement of eye characteristic while the presentation position Pp of the target changes.

The rate of change of the presentation position Pp can be set arbitrarily. In other words, the presentation position Pp of the target may change at a constant predetermined speed from the first presentation position Pp1 to the second presentation position Pp2, or the speed may change midway from the first presentation position Pp1 to the second presentation position Pp2.

In the example shown in FIG. 14, the presentation position of the target is changed continuously from the first presentation position Pp1 to the second presentation position Pp2, but in Step S23, the control portion 40 may change the presentation position of the target from the first presentation position Pp1 to the third presentation position Pp3 (see FIG. 9) at a position closer (- side) than the first presentation position Pp1. In other words, the presentation position Pp of the target should change continuously from the position set in Step S21 to an arbitrary position.

In Step S24, following the change of the presentation position Pp toward the second presentation position Pp2 in Step S23, the control portion 40 determines whether the flag for determining the end of the presentation of the target has turned on based on the measurement result of the objective measurement. If the control portion 40 determines YES (end determination flag = ON), it proceeds to Step S29, and if NO (end determination flag = OFF), it proceeds to Step S25. During the determination of the end determination flag ON, the control portion 40 displays the target on the subject eye E and continues to perform the objective measurement of the eye characteristic.

The turning ON/OFF of the flag for determining the end of the presentation of the target is determined based on the measurement result of the objective measurement after the presentation position Pp of the target has changed or while the presentation position Pp of the target changes. Here, for example, the end determination flag is turned on when either the 5th or 6th condition listed below is satisfied.
(Condition 5) The amount of change in the objective measurement value between the start of the presentation of the target and the determination of the flag for the end of the presentation of the target has reached a predetermined value. In other words, when it is determined that the objective measurement value at the time of the determination of the end determination flag changes a predetermined value (e.g. +3D) compared to the initial value.
(Condition 6) When the amount of change of the objective measurement value per a predetermined time while the presentation position Pp of the target changes is less than the threshold value. In other words, when it is determined that the slope of the change has decreased after convergence of the objective measurement values.

In this way, the turning ON/OFF of the flag for determining the end of the presentation of the target is determined based on the measurement result of the objective measurement after the presentation position Pp of the target has changed or while the presentation position Pp of the target changes, so that the end of the presentation of the target can be determined appropriately based on the changes in the objective measurement value of the eye characteristic associated with the changes in the presentation position Pp of the index. This allows the end of the presentation of the target to be determined appropriately based on the changes in the objective measurements of the eye characteristic associated with changes in the presentation position Pp.

In Step S25, following the determination that the end determination flag = OFF in Step S24, the control portion 40 determines whether the presentation position Pp of the target has aligned with the second presentation position Pp2 (target presentation position). When the control portion 40 determines YES (presentation position = second presentation position Pp2), it proceeds to Step S26, and if NO (presentation position *- second presentation position Pp2), it returns to Step S23.

In Step S26, following the determination in Step S25 that presentation position Pp of the target = second presentation position Pp2, the control portion 40 gradually changes the presentation position Pp of the target from the second presentation position Pp2 to the first presentation position Pp1, and proceeds to Step S27. Here, the control portion 40 continues to perform the objective measurement of the eye characteristics during the change in the presentation position Pp of the target.

In step S27, following the change of the presentation position Pp toward the first presentation position Pp1 in Step S26, the control portion 40 determines whether the flag for determining the end of the presentation of the target has turned on based on the measurement result of the objective measurement. When the control portion 40 determines YES (end determination flag = ON), it proceeds to Step S29, and when NO (end determination flag = OFF), it proceeds to Step S28. While the end determination flag is ON, the control portion 40 displays the target to the subject eye E with the presentation position Pp of the target fixed at the first presentation position Pp1, and continues to perform the objective measurement of the eye characteristic. The flag for determining the end of the presentation of the target is also turned on in Step S27 when either the fifth or sixth condition indicated in Step S24 is satisfied.

In step S28, following the determination that the end determination flag = OFF in Step S27, the control portion 40 determines whether the presentation position Pp of the target is aligned with the first presentation position Pp1 (target presentation position). When the control portion 40 determines YES (presentation position = first presentation position Pp1), it returns to Step S23, and when NO (presentation position ≠ first presentation position Pp1), it returns to Step S26.

In Step S29, following the determination that the end determination flag = ON in Step S24 or Step S27, the control portion 40 terminates the presentation of the target and determines the effect of the target recognition at the end of the target presentation, and proceeds to the end. The control portion 40 also terminates the objective measurement. In other words, the control portion 40 controls the operation of each part of the ophthalmologic apparatus 1 based on the determination that the end determination flag = ON in Step S24 or Step S27.

In the following, the relationship among the presentation position Pp of the target, the ON/OFF operation of the objective measurement, the objective refraction measurement value, and the end determination flag when the ophthalmologic apparatus 1 is controlled according to the flowchart shown in FIG. 14 will be described according to the time chart shown in FIG. 15.

At time t21 shown in FIG. 15, the control portion 40 presents the target at the first presentation position Pp1 (Step S21), and starts the objective measurement of the eye characteristic of the left and right subject eyes E (Step S22). As a result, at time t21, the presentation position Pp of the target changes from the OFF position (target not displayed) to the first presentation position Pp1, and the objective measurement operation changes from OFF to ON. Then, at time t21, the acquisition of the objective refraction measurement value begins. The end determination flag is set to OFF.

After presenting the target at the first presentation position Pp1 at the time t21, the control portion 40 continuously changes the presentation position Pp of the target from the first presentation position Pp1 to the second presentation position Pp2 (Step S23) with the target being presented. As a result, the subject eye E continues to visually recognize the target whose presentation position Pp gradually changes from the first presentation position Pp1 to the second presentation position Pp2. At this time, the objective measurement operation is maintained in the ON state, and the control portion 40 can continuously acquire the objective refraction measurement value of the subject eye E while viewing the target whose presentation position Pp is continuously changing.

After the time t21, the control portion 40 continuously monitors the amount of change in the objective refraction measurement value since the start of measurement and the amount of change in the objective refraction measurement value per predetermined time, etc., to determine whether the flag for determining the end of presentation of the target has turned on (Step S24) and to determine whether the presentation position Pp of the target matches the target presentation position, the second presentation position Pp2, which is the target presentation position (step S25), is performed repeatedly.

At the time t22, the presentation position Pp of the target coincides with the second presentation position Pp2, which is the target presentation position, and the control portion 40 determines that the presentation position Pp of the target has changed from the first presentation position Pp1 to the second presentation position Pp2 and that the end determination flag is OFF due to the reason that the objective refraction measurement value does not converge, for example, the control portion 40 gradually changes the presentation position Pp of the target from the second presentation position Pp2 to the first presentation position Pp1 (Step S26). At this time, the objective measurement operation remains ON, and the control portion 40 can continuously acquire the objective refraction measurement value of the subject eye E while viewing the target whose presentation position Pp changes continuously.

After the time t22, the control portion 40 continues to monitor the amount of change in the objective refraction measurement value since the start of measurement, the amount of change in the objective refraction measurement value per predetermined time, etc., and repeatedly determines whether the flag for determining the end of presentation of the target has turned on (Step S27) and whether the presentation position Pp of the target has matched the first presentation position Pp1 which is the target presentation position (Step S28). The determination as to whether or not the presentation position Pp of the target coincides with the first presentation position Pp1, which is the target presentation position (Step S28), is performed repeatedly.

At the time t23, the presentation position Pp of the target coincides with the first presentation position Pp1, which is the target presentation position, and the control portion 40 determines that the presentation position Pp of the target has changed from the second presentation position Pp2 to the first presentation position Pp1, and the end determination flag is OFF due to the reason that the objective refraction measurement value does not converge, for example, and the control portion 40 again gradually changes the presentation position Pp of the target from the first presentation position Pp1 to the second presentation position Pp2 (Step S23). At this time, the objective measurement operation remains ON, and the control portion 40 can continue to acquire the objective refraction measurement value.

Furthermore, after the time t23, the control portion 40 continues to monitor the amount of change in the objective refraction measurement value since the start of measurement and the amount of change in the objective refraction measurement value per predetermined time, etc., to determine whether or not the flag for determining the end of presentation of the target has turned on (Step S24), and to determine if the presentation position Pp of the target matches the first presentation position Pp1 which is the target presentation position. The determination as to whether or not the presentation position Pp of the target coincides with the first presentation position Pp1, which is the target presentation position (Step S25), is performed repeatedly.

At the time t24, when the control portion 40 determines that the flag for determining the end of the presentation of the target is ON, the control portion 40 terminates the presentation of the target and the objective measurement (Step S29).

As explained above, in the ophthalmologic apparatus of the present disclosure, when the control portion 40 presents a target on the target projection system 140, the presentation position Pp of the target may be continuously changed and the eye characteristic of the subject eye E may be measured by the objective measurement while the subject eye E is viewing the target whose presentation position Pp is continuously changed.

In the flowchart shown in FIG. 14, an example is shown in which the presentation position Pp of the target changes continuously from the first presentation position Pp1 to the second presentation position Pp2 and from the second presentation position Pp2 to the first presentation position Pp1. However, the presentation position Pp of the target may gradually change.

That is, in Step S31 of FIG. 16, the control portion 40 sets the presentation position Pp of the target to the first presentation position Pp1 near the far point of the subject eye E (see FIG. 9, for example, position shifted within ± 1D with respect to presentation position Pp (zero D) set in Step S1 in the first embodiment). The control portion 40 then moves the target unit 147 of the target projection system 140 along its optical axis to present the target at the first presentation position Pp1, and proceeds to step S32. In the example shown in FIG. 16, it is assumed that the presentation position Pp of the target is set to the first presentation position Pp1 in Step S31, but the control portion 40 can set the presentation position Pp of the target to any position in Step S31.

In Step S32, following the presentation of the target at the first presentation position Pp1 in Step S31, the control portion 40 starts the objective measurement of the eye characteristic of the left and right subject eye E, respectively, and proceeds to Step S33. The objective measurement of the eye characteristic is continued after Step S32.

In Step S33, following the start of the objective measurement in Step S32, the control portion 40 determines, based on the objective measurement value, whether or not the flag for determining the end of the target presentation has turned on. If the control portion 40 determines YES (end determination flag = ON), it proceeds to Step S38, and if NO (end determination flag = OFF), it proceeds to Step S34. The end determination flag for the presentation of the target is also turned on in Step S33 when either the fifth or sixth condition indicated in Step S24 is satisfied.

In step S34, following the determination that the end determination flag = OFF in Step S33, the control portion 40 determines whether a predetermined time has elapsed since the target was presented at the first presentation position Pp1. In other words, in Step S34, the control portion 40 determines whether or not the target has been continuously presented at the first presentation position Pp1 for the predetermined time. If the control portion 40 determines YES (predetermined time has elapsed), it proceeds to Step S35, and if NO (predetermined time has not elapsed), it returns to Step S31.

In Step S35, following the determination that the predetermined time has elapsed since the start of presentation of the target in Step S24, the control portion 40 changes the presentation position Pp of the target from the first presentation position Pp1 to the second presentation position Pp2 at a position farther (+ side) than the first presentation position Pp1 e.g., arbitrary position within +1D to +3D based on presentation position Pp (zero D) set in Step S1 in first embodiment). Then, the control portion 40 moves the target unit 147 of the target projection system 140 along its optical axis to present the target at the second presentation position Pp2, and proceeds to Step S36. In other words, in Step S35, the control portion 40 gradually changes the presentation position Pp of the target.

In the example shown in FIG. 16, the presentation position of the target is gradually changed from the first presentation position Pp1 to the second presentation position Pp2, but in Step S35, the control portion 40 may change the presentation position of the target from the first presentation position Pp1 to the third presentation position Pp3 (see FIG. 9), which is closer (- side) than the first presentation position Pp1 (see FIG. 9). In other words, the presentation position Pp of the target may be gradually changed from the position set in Step S31 to any position.

In Step S36, following the change of the presentation position Pp of the target in Step S35, the control portion 40 determines whether the end determination flag of the target presentation has turned on based on the measurement result of the objective measurement. If the control portion 40 determines YES (end determination flag = ON), it proceeds to Step S38, and if NO (end determination flag = OFF), it proceeds to Step S37. The end determination flag for the presentation of the target is also turned on in Step S36 when either the fifth or sixth condition indicated in Step S24 is satisfied.

In Step S37, following the determination that the end determination flag = OFF in Step S36, the control portion 40 determines whether a predetermined time has elapsed since the start of the presentation of the target at the second presentation position Pp2. In other words, in Step S37, the control portion 40 determines whether or not the target has been continuously presented at the second presentation position Pp2 for the predetermined time. If the control portion 40 determines YES (predetermined time has elapsed), it returns to Step S31, and if NO (predetermined time has not elapsed), it returns to Step S35. In the case of returning to Step S31, the presentation position of the target gradually changes from the second presentation position Pp2 to the first presentation position Pp1.

In Step S38, following the determination that the end determination flag = ON in Step S33 or Step S36, the control portion 40 terminates the presentation of the target and also determines the effect of the target recognition at the end of the target presentation, and proceeds to the end. The control portion 40 also terminates the objective measurement. In other words, the control portion 40 controls the operation of each part of the ophthalmologic apparatus 1 based on the determination that the end determination flag = ON in Step S33 or Step S36.

In the following, the relationship among the presentation position Pp of the target, the ON/OFF operation of the objective measurement, the objective refraction measurement value, and the end determination flag when the ophthalmologic apparatus 1 is controlled according to the flowchart shown in FIG. 16 will be described according to the time chart shown in FIG. 17.

At the time t31 shown in FIG. 17, the control portion 40 presents the target at the first presentation position Pp1 (Step S31) and starts the objective measurement of the eye characteristic of the left and right subject eye E (Step S32). As a result, at the time t31, the presentation position Pp of the target changes from the OFF position (target not displayed) to the first presentation position Pp1, and the objective measurement operation changes from OFF to ON. Then, at the time t31, the acquisition of objective refraction measurement value begins. The end determination flag is set to OFF.

After presenting the target at the first presentation position Pp1 at the time t31, the control portion 40 continuously measures the eye characteristic of the subject eye E with the target presented at the first presentation position Pp1. Therefore, the control portion 40 can continuously acquire the objective refractive measurement value of the subject eye E that is visually recognizing the target presented at the first presentation position Pp1.

After the time t31, the control portion 40 continuously monitors the amount of change in the objective refraction measurement value since the start of the measurement, the amount of change in the objective refraction measurement value per predetermined time, and the like, and repeatedly perform the determination whether or not the flag for determining the end of the target presentation has been turned on (Step S33) and the determination whether or not a predetermined time has passed since the target was presented at the first presentation position Pp1 (Step S34).

At the time t32, when a predetermined time has elapsed since the target was presented at the first presentation position Pp1, the control portion 40 changes the presentation position Pp of the target from the first presentation position Pp1 to the second presentation position Pp2 and has the target presented (step S35). At this time, the objective measurement operation is maintained in the ON state, and the control portion 40 can continue to acquire the objective refraction measurement value of the subject eye E when viewing the target at the presentation position Pp that has been changed.

After the time t32, the control portion 40 continues to monitor the amount of change in the objective refraction measurement value since the start of the measurement, the amount of change in the objective refraction measurement value per predetermined time, and the like, and repeatedly perform the determination whether the flag for determining the end of presentation of the target has been turned on (Step S36) and the determination whether or not a predetermined time has elapsed since the presentation of the target at the second presentation position Pp2 (Step S37).

At the time t33, when the control portion 40 determines that the predetermined time has elapsed since the target was presented at the second presentation position Pp2 and also determines that the end determination flag is OFF for a reason that the refraction measurement value has not converged, for example, the control portion 40 again changes the presentation position Pp to the first presentation position Pp1 and presents the Pp to the first presentation position Pp1 (Step S31). At this time, the objective measurement operation remains ON, and the control portion 40 can continue to acquire the objective refraction measurement value.

Furthermore, after the time 33, the control portion 40 continues to monitor the amount of change in the objective refraction measurement value since the start of the measurement, the amount of change in the objective refraction measurement value per predetermined time, etc., and repeatedly perform the determination whether the flag for determining the end of the presentation of the target has turned on (Step S33) and the determination whether the predetermined time has passed since the target was presented at the first presentation position Pp1 (Step S34).

At the time t34, if the control portion 40 determines that a predetermined time has elapsed since the target was presented at the first presentation position Pp1, and if the end determination flag is determined to be OFF for the reason that the objective refraction measurement value has not converged, for example, the control portion 40 again changes the presentation position Pp to the second presentation position Pp2 and the target is presented (Step S35). Then, the control portion 40 repeatedly determines whether or not the flag for determining the end of the presentation of the target has turned on (Step S36) and whether or not a predetermined time has elapsed since the target was presented at the second presentation position Pp2 (Step S37).

At the time t35, when the control portion 40 determines that the flag for determining the end of the presentation of the target is ON, the control portion 40 terminates the presentation of the target and terminates the objective measurement (Step S38).

As explained above, in the ophthalmologic apparatus of the present disclosure, when the control portion 40 presents a target on the target projection system 140, the presentation position Pp of the target may be gradually changed in steps, and the eye characteristic of the subject eye E may be objectively measured while the subject eye E is viewing the target whose presentation position Pp is gradually changing.

The control portion 40 may perform consecutively objective measurement several times after the target is viewed by the subject eye E for a predetermined period of time or while the subject eye is viewing the target. By performing consecutively objective measurement several times, it is possible to compare, for example, whether or not the amount of adjustment relaxation is greater than in the previous measurement.

An example of the specific control for performing consecutively objective measurement several times after or while the target is being viewed by the subject eye E is described below, based on the flowcharts shown in FIG. 18 or FIG. 20. Since the basic operation and control of each step is the same as in the first embodiment and the second embodiment, the detailed description will be omitted.

In Step S41, the control portion 40 sets the presentation position Pp of the target to an arbitrarily set predetermined presentation position Ppx. Then, the control portion 40 moves the target unit 147 of the target projection system 140 along its optical axis to present the target at the predetermined presentation position Ppx, and proceeds to Step S42.

In Step S42, following the presentation of the target in Step S41, the control portion 40 determines whether a predetermined time has elapsed since the target was presented at the predetermined presentation position Ppx. In other words, in Step S42, the control portion 40 determines whether the target has been continuously presented at the presentation position Ppx for the predetermined time. If the control portion 40 determines YES (predetermined time has elapsed), it proceeds to Step S43, and if NO (predetermined time has not elapsed), it returns to Step S41.

In Step S43, following the determination that the predetermined time has elapsed since the start of the presentation of the target in Step S42, the control portion 40 consecutively performs several times objective measurement of the eye characteristic of the left and right subject eyes E, respectively, and proceeds to Sep S44.

In Step S44, following the multiple implementation of the objective measurement in Step S43, the control portion 40 determines, based on the objective measurement value, whether or not the flag for determining the end of the target presentation has turned on. If the control portion 40 determines YES (end determination flag = ON), it proceeds to Step 45, and if NO (end determination flag = OFF), it returns to Step S43. The end determination flag for the presentation of the target is also turned on in Step S44 when either the fifth or sixth condition indicated in Step S24 is satisfied.

In Step S45, following the determination that the end determination flag = ON in Step S45, the control portion 40 terminates the presentation of the target and determines the effect of target recognition at the end of the target presentation and proceeds to the end. In other words, the control portion 40 controls the operation of each part of the ophthalmologic apparatus 1 based on the determination that the end determination flag = ON in Step S45.

In the following, the relationship among the presentation position Pp of the target, the ON/OFF operation of the objective measurement, and the end determination flag when the ophthalmologic apparatus 1 is controlled according to the flowchart shown in FIG. 18 will be explained according to the time chart shown in FIG. 19.

At the time t41 shown in FIG. 19, the control portion 40 presents the target at the predetermined presentation position Ppx (Step S41) and determines whether a predetermined time has elapsed since the target was presented (Step S42).

At the time t42, when the predetermined time has elapsed since the target is presented at the presentation position Ppx, the control portion 40 continuously measures the eye characteristic of the subject eye E several times with the target presented at the presentation position Ppx (Step S43). Then, the control portion 40 acquires a plurality of objective measurement values and determines whether or not the flag for determining the end of the target presentation has been turned on based on the multiple measurement results (Step S44).

Therefore, the control portion 40 can acquire a plurality of objective measurement values of the subject eye E after the target presented at the predetermined presentation position Ppx is viewed for a predetermined time. In addition, since the control portion 40 also presents the target during the objective measurement, it is possible to acquire a plurality of objective measurement values of the subject eye E while the subject eye E is viewing the target.

If, as a result of conducting the objective measurement several times at the time t43, the flag for determining the end of the presentation of the target is determined to be OFF, the control portion 40 continuously measures the eye characteristic of the subject eye E several times at the time t44, with the target again presented at the presentation position Ppx (Step S43). Then, the control portion 40 again determines whether or not the flag for determining the end of the presentation of the target has been turned on based on a plurality of measurement results (Step S44).

At the time t45, when the control portion 40 determines that the flag for determining the end of the presentation of the target is ON, the control portion 40 terminates the presentation of the target and terminates the objective measurement (Step S45).

As explained above, in the ophthalmologic apparatus of the present disclosure, after having the control portion 40 present a target for a predetermined time period, the control portion 40 may have multiple consecutive other-sensory measurements of eye characteristics be performed.

In the flowchart shown in FIG. 18, an example is shown in which the presentation position Pp of the target is fixed at the predetermined presentation position Ppx. However, the presentation position Pp of the target may change.

That is, in Step S51 of FIG. 20, the control portion 40 sets the presentation position Pp of the target to the first presentation position Pp1 near the far point of the subject eye E (see FIG. 9, for example, position shifted within ± 1D with respect to presentation position Pp (zero D) set in step S1 in first embodiment). The control portion 40 then moves the target unit 147 of the target projection system 140 along its optical axis to present the target at the first presentation position Pp1, and proceeds to Step S52. In the example shown in FIG. 20, it is assumed that the presentation position Pp of the target is set to the first presentation position Pp1 in Step S51, but the control portion 40 can set the presentation position Pp of the target to any position in Step S51. The control portion 40 can set the presentation position Pp of the target to any position in Step S51.

In Step S52, following the presentation of the target in Step S51, the control portion 40 determines whether a predetermined time has elapsed since the target was presented at the first presentation position Pp1. That is, in Step S52, the control portion 40 determines whether the target has been continuously presented at the first presentation position Pp1 for the predetermined time. If the control portion 40 determines YES (predetermined time has elapsed), it proceeds to Step S53, and if NO (predetermined time has not elapsed), it returns to Step S51.

In step S53, following the determination that the predetermined time has elapsed since the start of the presentation of the target in Step S52, the control portion 40 changes the presentation position Pp of the target from the first presentation position Pp1 to the second presentation position Pp2 at a position farther (+ side) than the first presentation position Pp1 (e.g., presentation position Pp set in Step S1 in first embodiment (e.g., arbitrary position within +1D to +3D based on position Pp (zero D) set in Step S1 in first embodiment). Then, the control portion 40 moves the target unit 147 of the target projection system 140 along its optical axis to present the target at the second presentation position Pp2 and proceeds to step S54. In other words, in Step S53, the control portion 40 gradually changes the presentation position Pp of the target.

In the example shown in FIG. 20, the presentation position of the target is gradually changed from the first presentation position Pp1 to the second presentation position Pp2, but in Step S53, the control portion 40 changes the presentation position of the target from the first presentation position Pp1 to the third presentation position Pp3 (see FIG. 9), which is closer (-side) than the first presentation position Pp1. In other words, the presentation position Pp of the target may be gradually changed from the position set in Step S51 to any position.

In Step S54, following the presentation of the target in Step S53, the control portion 40 determines whether a predetermined time has elapsed since the target was presented at the second presentation position Pp2. In other words, in Step S54, the control portion 40 determines whether the target has been continuously presented at the second presentation position Pp2 for the predetermined time. If the control portion 40 determines YES (predetermined time has elapsed), it proceeds to Step S55, and if NO (predetermined time has not elapsed), it returns to Step S53.

In step S55, following the determination that the predetermined time has elapsed since the start of the presentation of the target in Step S54, the control portion 40 performs multiple consecutive objective measurements of the eye characteristic of the left and right subject E, respectively, and proceeds to Step S56.

In Step S56, following the multiple implementation of the objective measurement in Step S55, the control portion 40 determines, based on the objective measurement value, whether or not the flag for determining the end of the target presentation has turned on. If the control portion 40 determines YES (end determination flag = ON), it proceeds to Step S57, and if NO (end determination flag = OFF), it returns to Step S51. The end determination flag for the presentation of the target is also turned on in Step S56 when either the fifth or sixth condition indicated in Step S24 is satisfied.

In Step S57, following the determination that the end determination flag = ON in Step S56, the control portion 40 terminates the presentation of the target and also determines the effect of the target recognition at the end of the target presentation and proceeds to the end. In other words, the control portion 40 controls the operation of each part of the ophthalmologic apparatus 1 based on the determination that the end determination flag = ON in Step S56.

In the following, the relationship among the presentation position Pp of the target, the ON/OFF operation of the objective measurement, and the end determination flag when controlling the ophthalmologic apparatus 1 according to the flowchart shown in FIG. 20 is explained according to the time chart shown in FIG. 21.

At the time t51 shown in FIG. 21, the control portion 40 presents the target at the first presentation position Pp1 (Step S51), and determines whether a predetermined time has elapsed since the target was presented at the first presentation position Pp1 (Step S52).

At the time t52, when the predetermined time has elapsed since the target was presented at the first presentation position Pp1, the control portion 40 changes the presentation position Pp of the target from the first presentation position Pp1 to the second presentation position Pp2 and presents the target (Step S53). Then, the control portion 40 determines whether or not the predetermined time has elapsed since the target was presented at the second presentation position Pp2 (Step S54).

When the predetermined time has elapsed since presenting the target at the second presentation position Pp2 at the time t53, the control portion 40 continuously measures the eye characteristic of the subject eye E several times with the target presented at the second presentation position Pp2 (Step S55). Then, the control portion 40 acquires the multiple objective measurement values and determines whether or not the flag for determining the end of the target presentation has been turned on based on the multiple measurement results (Step S56).

If the flag for determining the end of the presentation of the target is determined to be OFF as a result of the multiple objective measurements at the time t53, the control portion 40 again presents the target at the first presentation position Pp1 at the time t54 (Step S51). Then, when the predetermined time elapses at the time t55 (Step S52), the control portion 40 changes the presentation position Pp of the target to the second presentation position Pp2 and presents the target (Step S53).

When a predetermined time has elapsed at the time t56 (Step S54), the control portion 40 again measures the eye characteristic of the subject eye E several times in succession with the target presented at the second presentation position Pp2 (Step S55). Then, the control portion 40 acquires multiple objective measurement values and determines whether or not the flag for determining the end of the target presentation has been turned on based on the multiple measurement results (Step S56).

At the time t57, if the control portion 40 determines that the flag for determining the end of the presentation of the target is ON, the control portion 40 terminates the presentation of the target and terminates the objective measurement (step S57).

Thus, even when the presentation position Pp of the target changes, the control portion 40 can acquire multiple objective measurement values of the subject eye E after the target presented at the first presentation position Pp1 and the second presentation position Pp2 is viewed for a predetermined time. Since the control portion 40 also presents the target at the second presentation position Pp2 during the objective measurement, multiple objective measurement values of the subject eye E can be acquired while the subject eye E is being made to see the target.

Furthermore, in the case of multiple subjective measurements, the presentation position Pp of the target gradually changes in the example shown in FIG. 20, but the presentation position Pp of the target may change continuously, for example from the first presentation position Pp1 to the second presentation position Pp2.

The control portion 40 may determine that "the timing for the maximum cloud effect of the target viewing has passed" when the amount of adjustment relaxation no longer changes, or when the target position is moved a preset number of times (e.g., five times) and no increase in the amount of adjustment relaxation is observed two or more times, or the like.

As to the description of the above first, second embodiments and modified examples, the following is further disclosed.
(1) An ophthalmologic apparatus comprising:
   a target projection system that presents an eye chart to a subject eye;
   an objective measurement optical system that objectively measures an eye characteristic of the subject eye; and
   a control portion that controls the target projection system and the objective measurement optical system, wherein
   the control portion presents the target for a predetermined time at a predetermined presentation position with the target projection system, and objectively measures the eye characteristic with the objective measurement optical system in a time series to control an operation of each portion based on a measurement result of the objective measurement.
(2) The ophthalmologic apparatus according to the above (1),
   wherein the control portion sets the presentation position to a far presentation position farther than a far point of the subject eye, and presents the target at the far presentation position with the target presentation system to perform the objective measurement with the objective measurement optical system when the subject eye visually recognizes the target presented to the far presentation position.
(3) The ophthalmologic apparatus according to the above (1) or (2),
   wherein the control portion continuously or gradually changes the presentation position when presenting the target to the target projection system.
(4) The ophthalmologic apparatus according to the above (3),
   wherein the control portion presents a different target according to the presentation position when the target is presented to the target projection system.
(5) The ophthalmologic apparatus according to the above (3) or (4), wherein the control portion repeatedly changes the presentation position between a first presentation position near the far point of the subject eye and a second presentation position farther than the first presentation position when presenting the target to the target projection system.
(6) The ophthalmologic apparatus according to the above (3) or (4), wherein the control portion repeatedly changes the presentation position between a first presentation position near the far point of the subject eye and a third presentation position nearer than the first presentation position when presenting the target to the target projection system.
(7) The ophthalmologic apparatus according to any one of the above (1) to (6), wherein
   the target projection system presents a background image to a periphery of the target simultaneously with the target, and
   the control portion presents the target to the target projection system such that the subject eye is focused on the target, and the background image is burred.
(8) The ophthalmologic apparatus according to any one of the above (1) to (7), wherein
   the control portion alternately presents a wide angle image and a narrow angle image when presenting the target to the target projection system.
(9) The ophthalmologic apparatus according to any one of the above (1) to (8), wherein
   the target projection system presents a target to each of the left and right subject eyes, and
   the control portion controls the target projection system to provide parallax to the target when presenting the target to the target projection system.
(10) The ophthalmologic apparatus according to any one of the above (1) to (8), wherein
   the control portion sets the presentation position at a difference presentation position at which a visual line direction shifts in an up and down direction and/or a right and left direction relative to a planar front view, presents the target at the different presentation position with the target presentation position, and performs the objective measurement with the objective measurement optical system when the subject eye visually recognizes the target.
(11) The ophthalmologic apparatus according to the above (1), wherein
   the control portion continuously or gradually changes the presentation position when presenting the target to the target projection system, and intermittently objectively measures the eye characteristic with the objective measurement optical system while the subject eye visually recognizes the target in which the presentation position continuously or gradually changes.
(12) The ophthalmologic apparatus according to above (11), wherein
   the control portion controls operation of each portion based on a measurement result of the objective measurement during the presentation position changes or a measurement result of the objective measurement after the presentation position is changed.
(13) The ophthalmologic apparatus according to any one of the above (1) to (12), wherein
   the control portion performs the objective measurement of the eye characteristic with the objective measurement optical system several times after presenting the target for a predetermined time.

## Claims

1. An ophthalmologic apparatus comprising:
a target projection system (140) that presents a target to a subject eye (E);
an objective measurement optical system (160, 170) that objectively measures an eye characteristic of the subject eye (E); and
a control portion (40) that controls the target projection system (140) and the objective measurement optical system (10, 170), wherein
the control portion (40) presents the target for a predetermined time at a predetermined presentation position with the target projection system (140), and objectively measures the eye characteristic with the objective measurement optical system (160. 170) in a time series to control an operation of each portion based on a measurement result of the obj ective measurement.

2. The ophthalmologic apparatus according to claim 1,
wherein the control portion (40) sets the presentation position to a far presentation position farther than a far point of the subject eye (E), and presents the target at the far presentation position with the target presentation system (140) to perform the objective measurement with the objective measurement optical system (160, 170) when the subject eye visually recognizes the target presented to the far presentation position.

3. The ophthalmologic apparatus according to claim 1,
wherein the control portion (40) continuously or gradually changes the presentation position when presenting the target to the target projection system (140).

4. The ophthalmologic apparatus according to 3,
wherein the control portion (40) presents a different target according to the presentation position when the target is presented to the target projection system (140).

5. The ophthalmologic apparatus according to claim 3,
wherein the control portion (40) repeatedly changes the presentation position between a first presentation position near the far point of the subject eye and a second presentation position farther than the first presentation position when presenting the target to the target projection system (140).

6. The ophthalmologic apparatus according to claim 3,
wherein the control portion (40) repeatedly changes the presentation position between a first presentation position near the far point of the subject eye and a third presentation position nearer than the first presentation position when presenting the target to the target projection system (140).

7. The ophthalmologic apparatus according to claim 1, wherein
the target projection system (140) presents a background image to a periphery of the target simultaneously with the target, and
the control portion (40) presents the target to the target projection system (140) such that the subject eye is focused on the target, and the background image is burred.

8. The ophthalmologic apparatus according to claim 1, wherein
the control portion (40) alternately presents a wide angle image and a narrow angle image when presenting the target to the target projection system (140).

9. The ophthalmologic apparatus according to claim 1, wherein
the target projection system (140) presents a target to each of the left and right subject eyes, and
the control portion (40) controls the target projection system (140) to provide parallax to the target when presenting the target to the target projection system (140).

10. The ophthalmologic apparatus according to claim 1, wherein
the control portion (40) sets the presentation position at a difference presentation position at which a visual line direction shifts in an up and down direction and/or a right and left direction relative to a planar front view, presents the target at the different presentation position with the target presentation position, and performs the objective measurement with the objective measurement optical system when the subject eye visually recognizes the target.

11. The ophthalmologic apparatus according to claim 1, wherein
the control portion (40) continuously or gradually changes the presentation position when presenting the target to the target projection system (140), and intermittently objectively measures the eye characteristic with the objective measurement optical system while the subject eye visually recognizes the target in which the presentation position continuously or gradually changes.

12. The ophthalmologic apparatus according to claim 11, wherein
the control portion (40) controls operation of each portion based on a measurement result of the objective measurement during the presentation position changes or a measurement result of the objective measurement after the presentation position is changed.

13. The ophthalmologic apparatus according to claim 1, wherein
the control portion (40) performs the objective measurement of the eye characteristic with the objective measurement optical system (160, 170) several times after presenting the target for a predetermined time.
